# EUROPEAN PATENT SPECIFICATION

(11) **EP 2 293 761 B1**
(45) Date of publication and mention of the grant of the patent: **01.02.2017**
(21) Application number: 09746272.5
(22) Date of filing: 12.05.2009
(51) Int. Cl.: A61Q 17/04, A61Q 19/00, A61Q 19/02, A61Q 19/04, A61Q 19/06, A61Q 19/08, A61K 8/02, A61K 8/11

(54) **COMPOSITIONS FOR TOPICAL APPLICATION COMPRISING MICROENCAPSULATED COLORANTS**
MIKROVERKAPSELTE FÄRBEMITTEL UMFASSENDE ZUSAMMENSETZUNGEN ZUR TOPISCHEN ANWENDUNG
COMPOSITIONS POUR APPLICATION TOPIQUE COMPRENANT DES COLORANTS MICROENCAPSULÉS

(30) Priority: 12.05.2008 US 52411
(43) Date of publication of application: 16.03.2011
(73) Proprietor: Tagra Biotechnologies Ltd, 42293 Netanya (IL)
(72) Inventor: KVITNITSKY, Emma, 11632 Kiryat Shmona (IL); PALUY, Irena, 12235 Upper Galilee (IL); OLEINIK, Irena, 21033 Maalot (IL); SADE, Tal, IL-Maalot 21071 (IL); YASMAN, Yuri, 21996 Karmiel (IL)
(74) Representative: Gevers & Orès
(86) International application number: PCT/IL2009/000478
(87) International publication number: WO 2009/138978

(56) References cited:
- EP-A1- 1 908 452
- EP-A2- 1 175 895
- WO-A1-2007/083174
- WO-A2-01/35933
- WO-A2-2008/012709
- US-A- 5 753 244
- US-A1- 2006 051 425
- US-B1- 6 309 655

## Description

### FIELD OF THE INVENTION

The present invention relates to compositions for dermal/topical application comprising microencapsulated pigments. When applied to the skin, such compositions produce a color change indicating the delivery to the skin of the active substances contained in said compositions and a visual esthetic effect.

### BACKGROUND OF THE INVENTION

Compositions for topical applications comprising various colorants are known in the art. Previous attempts to use protected colorants in dermal applications were mostly focused towards hydrophobic or solid decorative cosmetics such as make-up, lipstick, blush, and powder products.

International Patent Application Publication WO 01/35933 discloses a method for microencapsulation of substances, by dissolving or dispersing the substances in an organic solvent that is partially water-miscible, mixing the organic solution with an aqueous solution which is saturated with an organic solvent to form an emulsion, and pouring the emulsion into water.

US Patent Application Publication No. 2006/0051425 discloses methods for microoencapsulating active ingredients for topical application within single-layer or multi-layer microcapsules, to protect the active ingredients, maintain their original activity, and enable controlled release only upon application onto the skin.

US Patents Nos. 5,320,835 and 5,382,433 disclose cosmetic formulations comprising a colored base phase, microcapsules comprising colorants, and colorant entrapping substrate particles dispersed in said base phase. The encapsulated colorants are said to be released into the base phase when mechanical action is applied to the cosmetic formulation, and produce an intense shade in the color of the base phase, whereas the colorant entrapping substrate particles entrap the released colorants and produce a subtle shade in the color of the base phase. The combined effect of the intense and the subtle shades produce a palette of color shades in the base phase, which is renewable upon application of mechanical action. The encapsulated pigments are made by a coacervation method. WO 98/5002 discloses similar color-sustainable base cosmetic formulations, further including volatile solvents to minimize the gritty feel of the microencapsulated material. The color obtained from the released encapsulated pigments is exactly the same as the color of the composition itself. Releasing provides renewed intensity of the original base color.

US 5,380,485 discloses colored cosmetic compositions, comprising particulate fillers coated with polymer that is combined with colorants, and their application in decorative cosmetics.

US Patent Application Publication Nos. 2005/0031558 and 2005/0276774 disclose a personal care or cosmetic composition containing microparticles comprising a shatter resistant blend of distinct colorants microencapsulated within a polymer matrix, preferably a cross-linked polymer matrix that does not allow any of the entrapped colorant to be released even under prolonged use. The matrix polymer is preferably transparent or translucent such that the blend of encapsulated colorants provides the coloring of the cosmetic product itself and of the skin upon application of the cosmetic composition. The microparticles disclosed in 2005/0276774 further contain secondary particles (i.e. hydrophobic polymers different from those of the matrix polymer) that are distributed throughout the matrix.

US Patent Application Publication No. 2005/0265938 discloses decorative color cosmetic compositions containing different pigment types and a carrier, wherein the different pigment types are physically separated from each other in individual capsules (silica capsules or micelles) within the carrier. The pigments remain separate and distinct in the final product and on the skin. The color of the composition is determined by the relative amounts of the different pigments and remains the same upon application to the skin.

US Patent Application Publication No. 2006/0093564 discloses colored cosmetic composition comprising rupturable spherules filed with colorant that, when applied to the skin, the colorant impregnated within the spherules is released into the skin. The emptied spherules further have an uptake function; namely, serve as a collection device for skin exudates such as sebum or perspiration.

There is a growing interest in cosmetic products that provide a change in color in response to external incentives such as pH-change, shear force, light, heat, and the like.

US Patent No. 4,756,906 discloses decorative cosmetic compositions containing a first colorant and microcapsules containing a solvated second colorant, different from the first colorant. Upon rupture of the microcapsules, the coloration of the encapsulated pigment is added into the composition thereby altering its color characteristics.

US Patent No. 6,309,655 discloses non-oil based anhydrous cosmetic compositions comprising self-heating components (generate heat when brought into contact with water), and self-indicating disintegrating granules, comprising water-insoluble polymer and colorants. The colorants are released from the granules under physical manipulation and provide a color change, which gives the user an indication of the length of time of application and of effective mixing.

US Patent No. 6,733,766 discloses a substantially dry composition comprising a non-capsulated colorant activable by water to reveal a visual color, and an oil-soluble carrier. The colorant is insoluble in the carrier and the visual color is not imparted to the composition in its dry state. Addition of water to the composition activates a color change thus indicating the wetting of the composition.

US Patent Application Publication 2006/0057084 discloses color-changing compositions comprising color aggregates, which comprise anionic polymers and optionally surface treated non-encapsulated metal oxide colorants, non-covalently, preferably ionically (e.g. salt formation), associated with the polymer. Upon application of chemical energy to the aggregate, the colorants dissociate from the anionic polymer and produce a visible color change to the composition at the location of application.

US Patent Application Publication No. 2006/0067896 discloses a self-indicating sunscreen or sunblock system of complementary reagents which comprises a sunscreen formulation containing a color-triggering developer and a marking composition, which is a formulation containing a color precursor capable of reacting with the color-triggering developer after application of the composition to the skin, to produce a color.

US Patent No. 5,753,244, which represents the closest state of the art to the present invention, discloses a product for topical application comprising a microencapsulated color-disappearing or color-changing indicator, which disappears or changes color after a short period of time following application to the skin and upon exposure to air, heat, light or skin acids.

### SUMMARY OF THE INVENTION

The present invention relates to a color-changing composition for skin care according to claim 1 comprising one or more active substances and one or more microencapsulated colorants and optional non-encapsulated one or more colorants wherein, upon application on the skin, the composition provides a changing color effect that indicates the delivery of the active substances from said composition onto the skin and/or gives to the skin a visual esthetical effect.

The compositions of the invention are for topical application and may be cosmetic or therapeutic compositions for skin care.

### DETAILED DESCRIPTION OF THE INVENTION

The present invention relates to a composition according to claim 1.

The present invention relates to functional/multifunctional color-changing cosmetic compositions comprising biologically and/or therapeutically active substances for topical use, which upon application, e.g. rubbing onto the skin, immediately and irreversibly change their color. This change of color occurs in the skin area covered by the composition and indicates the delivery of active materials to the applied skin area. In addition, the change of color generated a visual esthetic effect.

The change of color is provided by the colorant-containing microcapsules, which upon rupture by application of a mechanical force, release the entrapped colorant into the composition, thereby changing its color. A mechanical action such as rubbing spread the topical composition and facilitates its penetration into the skin. The immediate change of color of the composition provides a desirable esthetic effect and, moreover, enables the user to assess the skin area applied with the desired biologically and/or therapeutically active agent.

The terms "functional" and "multifunctional effect" as used herein refers to effects produced by active substances in the cosmetic products or cosmetic compositions that may provide health benefits for the skin beyond basic esthetic or decorative effects.

The term "microcapsule", as used herein, refers to a spherical microparticle consisting of a polymeric shell serving as a wall-forming material and an encapsulated material, e.g. an active substance or a colorant, located within the core of the microcapsule. Microcapsules are distinct from microspheres, which consist of spherical homogeneous granules of the active substance dispersed in a polymer and are, in strict sense, spherically empty particles.

The term "single-layer microcapsule" refers to a microcapsule consisting of a single polymeric shell and an encapsulated colorant or active substance located within the core of the microcapsule.

The term "inner core microcapsule" refers to a single-layer microcapsule as defined above when it is located within a single-layer or multi-layer microcapsule.

The term "double-layer microcapsule" refers to a microcapsule consisting of the inner core microcapsule coated with a second polymeric shell that may be identical or different from the first polymeric shell.

The term "multi-layer microcapsule" refers to a microcapsule consisting of an inner core microcapsule and one or more outer polymeric shells. The one or more outer shells of the multi-layer microcapsule and the single layer of the inner core microcapsule may be comprised of the same or different wall-forming polymer(s).

The term "wall-forming polymer" refers to a polymer or a combination of two or more different polymers as defined herein, which form a component of the external wall or layer or shell of the microcapsules.

The term "polymer shell" refers to a polymer layer containing the wall-forming polymer and, optionally, further components such as a plasticizer and/or a mineral. The term "polymer-plasticizer shell" refers to a polymer shell containing a plasticizer. The term "polymer-mineral shell" refers to a polymer shell containing a mineral.

The terms "composite double-layer" or "composite multi-layer microcapsule" refer herein to a microcapsule in which the inner or outer shell is a polymer-mineral shell.

The terms "colorant", "color agent" and "pigment" are used herein interchangeably and refer to organic pigments such as synthetic or natural dyes selected from any of the well known FD&C or D&C dyes, inorganic pigments such as metal oxides, or lakes and any combination (blend) thereof. In preferred embodiments, the color agent is an inorganic pigment, more preferably a metal oxide.

The terms "active agent", "active", "active ingredient", or "active substance", are used herein interchangeably and refer to natural or synthetic substances that may have one or more biological and/or therapeutic activities. These terms also include fragrances and perfumes as well as cooling and warming agents.

The term "therapeutic agent" is used herein for a natural or synthetic substance that provides one or more therapeutic properties to the cosmetic compositions of the invention.

The term "natural extract" refers herein to ingredients of botanical origin, which did not undergo any significant chemical changes. This term includes plant oils such as essential oils.

The terms "topical application" and "dermal application" are used herein interchangeably and refer to external application onto the skin.

The term "compositions for topical application" includes compositions in any form such as ointment, paste, cream, gel or lotion intended for skin care, skin supplement, sun care, baby care, pharmaceutical compositions for dermal application, and similar compositions.

Thus, in one aspect, the present invention provides a color-changing cosmetic composition for skin care comprising one or more active substances and one or more microencapsulated colorants and optional non-encapsulated one or more colorant wherein, upon application on the skin, the composition provides a changing color effect, which indicates the delivery of the active substances from said composition onto the skin and/or gives to the skin a visual esthetical effect.

The colorant useful according to the present invention may be oil-soluble or oil-dispersible or with limited solubility in water. Typically suitable colorants include organic and inorganic pigments, lakes, natural and synthetic dyes and any combination thereof.

In some preferred embodiments, the color agents are inorganic pigments such as, but not limited to, metal oxides such as iron oxides, titanium dioxide (TiO₂), titanium lower oxides, aluminum oxide, zirconuim oxides, cobalt oxides, cerium oxides, nickel oxides chromium oxide (chromium green), zinc oxide and composite metal oxides; metal hydroxides such as calcium hydroxide, iron hydroxides, aluminum hydroxide, chromium hydroxide, magnesium hydroxide and composite metal hydroxides; other colorants such as ferric ammonium ferrocyanide, Prussian blue, iron sulfides, manganese violet, carbon black, mica, kaolin, and mixtures thereof.

In more preferred embodiments, the inorganic pigments are selected from iron oxides, titanium dioxide, zinc oxide, chromium oxide/hydroxide, and mixtures thereof. In a more preferred embodiment, the color agent is iron oxide of any one of the three primary colors - red, yellow or black, or most preferably, a mixture thereof. Optionally, the colorant may comprise, besides the mixture of iron oxides, titanium dioxide, for the purpose of providing any desired final color or shade of color to the composition. Preferably, titanium dioxide is used in any one of its mineral forms such as, but not limited to, anatase, brookite or rutile, or mixtures thereof.

In another embodiment, the colorants are Lake organic pigments produced by precipitation of a natural or synthetic dye with a metallic salt such as aluminum, calcium or barium salts. They are oil-dispersible and widely used in cosmetics. Examples of Lake pigments useful for the purpose of the invention include, but are not limited to, Indigo Lakes, Carmine Lakes, lakes from the series of the well-known FD&C and D&C dyes such as D&C Red 21 Aluminum Lake, D&C Red 7 Calcium Lake. In a preferred embodiment, the colorant is a Carmine Lake.

In another embodiment, the color agent is a natural or synthetic organic dye, for example, aromatic azo, indigoid, triphenylmethane, anthraquinone and xanthine dyes of the well-known D&C and FD&C series.

According to the present invention, at least one of the colorants in the composition is microencapsulated and additional colorants may be microencapsulated or non-encapsulated.

The color-changing compositions of the invention may comprise microcapsules containing only one type of pigment or a mixture of two or more pigments, either encapsulated individually and/or one or more blends of colorants may be encapsulated within the core of single-, double- or multi-layer microcapsules. A person skilled in the art will know how to choose pigments and combinations of pigments to produce a desired color effect or color change.

The active substances may be organic or inorganic, natural or synthetic substances such as, but not limited to, vitamins, natural extracts, essential oils, individual compounds prepared synthetically or isolated from a natural source, including volatile natural and synthetic compounds, and pharmaceutical agents for topical application.

The active substances possess biological and/or therapeutic activity or multiple activities essential for skin care. The one or more functional/multifunctional active substances are selected from moistening, healing/regenerating/revitalizing, depigmentation/whitening/lightening, antioxidant/radical scavenger, cooling/calming, warming, anesthetic, sunscreen/sunblock, sunless tanning, antibiotics, anti-cellulite, keratolytic, antifungal, antipsoriatic, anti-inflammatory, antibacterial, astringent, antiseptic, repellent, anti-spider vein, anti-rosacea/anti-couperosis, anti-acne agents, fragrances/scents and agents for treatment of fever blisters.

The active substances may be microencapsulated or non-encapsulated. Preferably, sensitive or unstable active substances will be microencapsulated. For example, active agents such as vitamins, oils, natural extracts, essential oils, odor agents such as fragrances as well as pharmaceuticals for topical application such as antibiotics, are microencapsulated and effectively protected within single-layer microcapsules. More preferably, active agents such as vitamins, natural extracts, essential oils, individual compounds isolated from natural sources and therapeutic agents for topical application are encapsulated within double- or multi-layered microcapsules.

In one embodiment, the active substance with beneficial properties for the skin is a natural extract such as any herbal extract or plant oil used in topical applications, including essential oils. The herbal extracts or plant oils are preferably microencapsulated.

Preferred herbal extracts and plant oils include, but are not limited to: Licorice root extract, a whitening and anti-spot agent known for inhibiting tyrosinase and melanin synthesis and useful for treatment of age spots, pigmentation during pregnancy, post-inflammatory hyperpigmentation; Grape Seed extract, an antioxidant/free radical scavenger, sunscreen/sunblock, and antifungal agent that, when encapsulated, maintains its supreme anti-oxidant activity; Borage oil, a moisturizing, calming, antioxidant/free radical scavenger and anti-inflammatory agent; Jojoba oil, a moistening anti-inflammatory agent; Evening Primrose oil, a moisturizing, calming, regenerating/revitalizing and antioxidant/free radical scavenger agent; Hippophae/Sea Buckthorn Oil, containing stable high concentration of multivitamins and carotenoids and is beneficial as skin regenerator, calming and anti-inflammatory; Tea Tree oil, a natural antibacterial, antiseptic, antifungal, healing/regenerating/revitalizing and anti-inflammatory agent useful in the treatment of acne, psoriasis, vaginitis, etc.; Carrot Seed extract and oil, useful as healing/regenerating/revitalizing agent; Camellia Sinensis leaf extract (Green Tea or Black Tea) useful as antioxidant agent; Chamomile Oil, provides soothing and calming properties as well as anti-inflammatory effects; Ginger Oil contains high anti-oxidant and anti-inflammatory properties; Eucalyptus Citriodora Oil, provides calming, antifungal and anti-inflammatory properties; and mixtures thereof.

In other embodiments, the active substance with beneficial properties for the skin are vitamins, e.g., the vitamins A, B, C, D, E, F, K, P, or mixtures thereof. In preferred embodiments, the composition of the invention comprises microcapsules containing one or more of following vitamins: vitamin A, either in its free form as Retinol or in its ester form as Retinol Palmitate, useful as skin regenerating, whitening/lightening, keratolytic, anti-acne, anti-psoriatic and anti-inflammatory agent; vitamin C (ascorbic acid) and its derivatives, useful as anti-oxidant/free radical scavenging, whitening/lightening and anti-psoriatic agents; vitamin E, preferably as α-tocopherol, useful as anti-oxidant/free radical scavenging, and anti-inflammatory agent; vitamin F, a mixture of unsaturated linoleic and alpha-linolenic fatty acids, also known as Essential Fatty Acids (EFA), essential for skin health and functionality as moisturizing, cooling/calming, anti-oxidant/free radical scavenging and anti-inflammatory agent; Rutin (quercetin-3-Rutinoside or vitamin PI) and Rutin Hydrate, one of the most active natural flavonoids, an antioxidant/free radical scavenger, anti-cellulite and anti-inflammatory agent.

In a further embodiment, the active substance is a pharmaceutical agent suitable for dermal applications, selected from an antibiotic such as, but not limited to, erythromycin, azithromycin or clarithromycin, or any of the moisturizing, whitening/lightening, depigmentation, antioxidant/free radical scavenger, cooling/calming, keratolytic, astringent, healing/regenerating/revitalizing, rejuvenating, anti-cellulite, anti-spider vessel, anti-acne, and anti-psoriatic agents as will be defined hereinafter.

According to their functionality, the active substances for use in the compositions of the invention may be classified in the following categories:
(i) moistening or moisturizing agents such as, but not limited to, Evening Primrose oil, Borage oil, Jojoba oil, Aloe vera gel, Vitamin F, panthenol, and mixtures thereof;
(ii) healing/revitalizing/regenerating agents such as, but not limited to, Hippophae (Sea Buckthorn) oil, Tea Tree oil, vitamin A, allantoin and derivatives, carotenoids, Carrot Seed extract and oil, Patchouli essential oil, and mixtures thereof
(iii) depigmentation (whitening)/lightening agents such as, but not limited to, Licorice (Gycyrrhiza Glabra) root extract, arbutin, kojic acid, hydroquinone, beta-hydroxy acids such as salicylic acid, alpha-hydroxy acids, vitamin C and derivatives, and mixtures thereof;
(iv) antioxidant/free radical scavenging agents such as, but not limited to, vitamin E, tocotrienols, tocopherols, vitamin F, vitamin C and derivatives thereof, Rutin, Resveratrol and derivatives thereof, Camellia Sinensis leaf extract (Green Tea or Black Tea), Grape Seed extract, Evening Primrose oil, Borage oil, Ginger essential oil, curcumin, chitosan, carotenoids, and mixtures thereof;
(v) cooling and calming agents such as, but not limited to, menthol, Aloe Barbadensis leaf extract, camphor, methyl salicylate, menthyl lactate, allantoin, bisabalol, Chamomile extract and essential oil, Evening Primrose oil, Borage oil, Eucalyptus Citriodora essential oil, Patchouli essential oil, panthenol, and mixtures thereof;
(vi) warming agents such as, but not limited to, black pepper extract and essential oil, paprika (red pepper) extract, Cinnamon extract and essential oil, Ginger root extract and essential oil, zeolite; and mixtures thereof;
(vii) anesthetic agents such as, but not limited to, Aloe barbadensis gel (Aloe vera gel), benzocaine, lidocaine, dibucaine, pramoxine, tetracaine, camphor, resorcinol, and mixtures thereof;
(viii) sunscreen/sunblock agents such as, but not limited to, p-aminobenzoic acid (PABA) and esters thereof, benzalphthalides, benzophenones, cinnamates, etocrylene, octocrylene, salicylates, Grape Seed extract, titanium dioxide, zinc oxide, and mixtures thereof;
(ix) sunless tanning agents such as, but not limited to, 1,3-dihydroxyacetone, melanin, mahakanni, erythrulose, 5-hydroxy-1,4-naphthoquinone, 5-hydroxy-1,4-naphthoquinone, and mixtures thereof;
(x) antibiotics for topical application such as, but not limited to, erythromycin, clarithromycin, azithromycin and clindamycin;
(xi) anti-cellulite agents such as, but not limited to, Rutin hydrate, xanthine, caffeine, theophilline, theobromine, aminophylline, and mixtures thereof;
(xii) keratolytic agents useful for treating acne, warts and other skin diseases such as, but not limited to, salicylic acid and other beta-hydroxy acids, alpha-hydroxy acids such as glycolic acid, benzoyl peroxide, sulfur, isotretinoin, tretinoin, aminolevulinic acid, fluorouracil, podophyllotoxin, podophyllum, propylene glycol, phytic acid, and mixtures thereof;
(xiii) antibacterial agents such as, but not limited to, antibiotics, Chamomile essential oil;
(xiv) antifungal agents such as, but not limited to, Tea Tree oil, Grape Seed extract, Eucalyptus Citriodora essential oil, ursolic acid, essential oils, amphotericin, itaconazole, fluconazole, ketoconazole, miconazole, morpholine, undecylenic acid, and mixtures thereof;
(xv) anti-acne agents such as, but not limited to, benzoyl peroxide, tretinoin, isotretinoin, Chamomile essential oil, and mixtures thereof;
(xvi) antipsoriatic agents such as, but not limited to, retinoids, vitamin A, vitamin C, vitamin D3 and analogs thereof, ergocalciferol, carotenoids, anthralin, metronidazole, tazarotene, cyclosporine, pyrogallol, allantoin, and mixtures thereof;
(xvii) anti-inflammatory agents such as, but not limited to, vitamin F, vitamin E, carotenoids, vitamin A, unsaturated fatty acids, Rutin, bioflavanoids, Hippophae (Sea Buckthorn) oil, Olive oil, Salicin, Ginger root extract and essential oil, Jojoba oil, Chamomile essential oil, Eucalyptus Citriodora essential oil, ursolic acid, triamcinolones, cortisones, prednisones, cortodoxone, flucetonide, medrysone, amcinafel, amcinafide, betamethasone and esters thereof, clocortelone, descinolone, desonide, flucloronide, flumethasone, flunisolide, fluocinonide, flucortolone, paramethasone, dexamethasone, fluroandrenolone acetonide, acetonide, dichlorisone, and mixtures thereof;
(xviii) astringent agents such as, but not limited to, Witch hazel (Hamamalis Virginiana), Yarrow (Achillea millefolium), Rosewood oil, benzoin, aluminum sulfate and other aluminum salts, salicylic acid, zinc oxide, and mixtures thereof;
(xix) antiseptic agents such as, but not limited to, honey, curcumin, captan, chlorhexidine and its derivatives, hexachlorophene, triclosan, triacetin, sodium usnate, sulfur, and mixtures thereof;
(xx) repellent agents such as, but not limited to, essential oils, Pyrethrin, Permethrin, Bioresmethin, dimethylphthalate, and mixtures thereof;
(xxi) antirosacea agents such as, but not limited to, Vitamin K, sulfur, Marigold oil, Rutin, bioflavonoids, and mixtures thereof;
(xxii) active substances beneficial for fever blisters (cold sores and shingles) local treatment including, but not limited to, acyclovir, antihistamines, local anesthetic, essential oils possessing antiviral activity, and mixtures thereof; and
(xxiii) fragrances/scents including, but not limited to, lavender, Neroli fragrance oil, Chamomile essential oil, Ginger essential oil, Patchouli essential oil, Eucalyptus Citriodora essential oil, Rosemary essential oil, Sandalwood essential oil, Tea Tree oil, and mixtures thereof.

The most preferred cooling agent is microencapsulated menthol, which provides the cosmetic formulations with a fresh sensation, cooling effect, calming qualities and short-term relief. Microcapsules containing 10% menthol are used according to the present invention in skin care products selected from sunscreen products, cooling after-sun lotions, calming creams and refreshing pre- and aftershave products.

Sunscreens are important skin-care products used to prevent photoaging and skin cancer. There are two groups of sunscreens: UVA sunscreens, which block UV radiation in the wavelength range of about 320 to 400 nm, and UVB sunscreens, which block radiation in the range of 290 to 320 nm. Sunscreen compositions that contain mixtures of UVA and UVB type sunscreen actives may provide an SPF (sun protection factor) of from 2 to 50.

The present invention intends to encompass active substances and colorants approved by the Personal Care Products Council (formerly Cosmetic, Toiletry and Fragrances Association, CTFA) and Food and Drugs Administration (FDA). In addition, according to requirements of the cosmetic industry, the microcapsules of the present invention do not contain ethanol.

Any suitable microencapsulation method can be used according to the present invention. In most preferred embodiments, the microencapsulation method is based on the solvent removal method as described in U.S. Patent No. 6,932,984 and U.S. Patent Application No.11/208,007 (Publication US 2006/0051425), both documents herewith incorporated by reference as if fully disclosed herein. This method has a universal application and in the cosmetic and pharmaceutical industries may be applied for encapsulation of oil-soluble and oil-dispersible substances and substances with limited solubility in water.

Thus, single-layer or multi-layer microcapsules for use in the compositions of the present invention, encapsulating in their core one or more colorant or one or more active ingredient, and comprising one or more shells of the same or different wall-forming polymer, are produced by a method comprising the steps of:
(a) preparing an organic solution comprising: (i) an active agent/colorant dissolved or dispersed therein; (ii) a wall-forming polymer selected from the group consisting of a polyacrylate, a polymethacrylate, low molecular weight (about 15,000 D) poly(methyl methacrylate)-*co*-(methacrylic acid) (1:0.16), poly(ethyl acrylate)-*co*-(methyl methacrylate)-*co*-(trimethylammmonium-ethyl methacrylate chloride) (1:2:0.1), poly(butyl methacrylate)-*co*-(2-dimethylaminoethyl methacrylate)-*co*-(methyl methacrylate) (1:2:1), poly(styrene)-*co*-(maleic anhydride), copolymer of octylacrylamide, cellulose ethers, cellulose esters and poly(ethylene glycol)-*block*-poly(propylene glycol)-*block-*poly(ethylene glycol); (iii) an organic solvent of a kind that is partially miscible with water and is capable of dissolving or dispersing the substances of (i) and (ii); and, optionally, (iv) an antioxidant, a plasticizer or both;
(b) preparing an aqueous continuous phase saturated with said organic solvent and comprising an emulsifier;
(c) while agitating, pouring the organic solution or dispersion of (a) into the aqueous continuous phase of (b) to form an emulsion;
(d) adding an excess amount of water to the emulsion obtained in (c) to initiate extraction of the organic solvent from the emulsion, and continuing the extraction by incubating the solvent, thus promoting the formation of solid single-layer microcapsules (hereinafter "the core microcapsules");
(e) isolating the core microcapsules, washing with water or an aqueous solution of alcohol and drying them, thus obtaining single-layer microcapsules; and, optionally
(f) forming multi-layer microcapsules by treating the surface of the dried core single-layer microcapsules of (e) with a material that modifies the morphology of the core surface, increases its specific surface area and facilitates the adhesion of an additional polymeric shell, and either repeating steps (a) to (e) to form double-layer microcapsules, or repeating steps (a) to (f) followed by steps (a) to (e) one or more times to add two or more additional layers surrounding the core microcapsule.

The encapsulation method of the present invention masks the original color of the encapsulated colorants, increases the stability of sensitive substances against degradation, and prevents undesirable release of the encapsulated substances into the composition during the manufacturing process and prolonged storage.

Encapsulation of the colorants prevents undesirable re-agglomeration of pigments during manufacture and prolonged storage of the cosmetic products. Encapsulation of colorant also enables the incorporation, into a single composition, of actives and colorants, which are incompatible with each other and for which a direct contact might be detrimental. In addition, microencapsulation of the colorants allows the use of regular equipment for the preparation of the compositions of the invention because no coloring of the apparatus occurs during the manufacturing process.

Suitable microcapsules containing the colorant or active agents according to the invention, should be capable of swelling but at the same time maintain the impermeability of the capsules' wall and prevent the release of the encapsulated substances into the formulations. These microcapsules should be soft enough to rupture upon very slight rubbing or pressing on the skin in order to release their content but, nevertheless, should be durable enough to avoid destruction of the shell and realization of content during their isolation, drying or sieving.

The consumer applying a composition of the invention can sense the differences before and after application via different sensory signals: the vision/sight signal provided by the change of color of the composition upon application, the olfaction/smell signal provided by the release of microencapsulated fragrances/scents, and the skin sensation provided by the delivery of microencapsulated cooling or warming agents.

The multi-layer microcapsules may be formed form the same or different wall-forming or shell-forming polymers, but they all possess the same physicomechanical characteristics, are both water and oil insoluble, thermostable, not fragile and not rupturable while handling. Such microcapsules are effective in the masking of colors and malodor and in the protection of unstable, sensitive and/or volatile substances.

The effectiveness of protection/masking by a single-layer microencapsulation depends on the chemical structure, molecular weight and physical properties of the encapsulated substance. For some colorants and active substances, single-layered microencapsulation would not provide an adequate masking effect and/or protection from degradation. For such substances a double- or multi-layered microencapsulation is employed.

In one preferred embodiment, the microcapsules are composite double- or multi-layered spherical particles with a size of less than 70 µm, consisting of an inner core containing the colorant or active and one or more outer shells of the same or different wall-forming polymer.

In a more preferred embodiment, the polymer shell contains a plasticizer such as tricaprylin, trilaurin, tripalmitin, triacetin, triethyl citrate, acetyltriethyl citrate, isopropyl myristate, paraffin oil, or a mixture thereof, in order to control the physical properties and level of elasticity of the microcapsules.

The compositions and cosmetic products prepared therefrom may contain additional natural or synthetic dyes in a non-encapsulated form to provide a desired initial color to the product before its application. This initial color may be totally different from the color, which eventually develops on the skin upon release of the encapsulated colorants.

The color-changing compositions of the present invention may be prepared by conventional procedures known in the art of manufacture of compositions for topical application. In one embodiment, the compositions are prepared by simple physical blending of suitable encapsulated colorants and encapsulated or non-encapsulated active substances into skin care formulations.

In a more preferred embodiment, the cosmetic composition is prepared by blending of at least three separately microencapsulated colorants, preferably metal oxides, more preferably iron oxides, wherein each of said colorants comprises at least one primary colorant.

The color-changing cosmetic and therapeutic compositions of the invention may contain, besides the colorants and active ingredients mentioned above, excipients well known in the art such as emulsifiers, emollients, thickeners, surfactants, chelating agents, gelling agents, deionized water, preservatives, humectants, consistency factors, chelating agents, fragrances, aesthetic enhancers, skin penetration enhancers, exfoliants, lubricants, and pH regulators.

The color-changing cosmetic composition of the invention may be formulated as oil-in-water, oil-in-water-in-oil, water-in-oil, or water-in-oil-in water emulsions, aqueous formulations or anhydrous formulations.

The cosmetic and therapeutic products of the invention are preferably in the form of cream, lotion, gel, milk, balm, mask or make-up form. In one preferred embodiment, the product is a cream, more preferably a facial cream, balm or lotion that is an oil-in-water emulsion. In another preferred embodiment, the product is a mask.

In more preferred embodiments of the invention the cosmetic or therapeutic compositions possesses multifunctional activities and selected from, but not limited to, body skin care, facial skin care, baby care, sunscreen care, after sun, sunless tanning, whitening, aftershave, anti-acne, anti-repellent, anti cellulite and anti-perspirant compositions.

In one preferred embodiment, a multifunctional color-changing sunscreen/sun-block composition is provided, having the indicative color effect of product application and antioxidant activity. Old-fashion sun screening/sun-blocking compositions provide UV light protection as a primary shield. Today's sunscreens are balanced sunscreen protection systems that provide two defense mechanisms: filtering UV rays and antioxidant activity to neutralize reactive oxygen species (ROS) as a secondary shield. The non-delayed release of colorants from the ruptured microcapsules and spread of color shade on the skin upon application, guarantee that all desirable skin areas applied are covered with the sunscreen and protected from UV radiation. The unpleasant whitening of the applied skin area, characteristic of known sunscreen/sun-block products, is prevented when applying the multifunctional color-changing sunscreen composition of the present invention. In addition, the present compositions confer high stability to relatively unstable active ingredients such as organic UV absorbers, and provide the aesthetic benefit of a fresh tan/bronze skin tone immediately upon application. Moreover, the microencapsulated inorganic pigments dramatically increase the efficiency of radiation protection due to the ability of inorganic pigments to acts as UV absorbers, particularly metal oxide colorants such as iron oxides, titanium dioxide and zinc oxide, which increase the optical path length. The action of the microencapsulated colorants as UV absorbers is possible due to the use of polymers that are clear for UV light.

In accordance with this specific embodiment, the multifunctional color-changing sunscreen composition comprises one or more, preferably three or more colorants, preferably separately microencapsulated colorants, and sunscreen agents, preferably both organic and inorganic sunscreen agents, an antioxidant/radical scavenger and a moistening agent in a microencapsulated or non-encapsulated form, wherein the color agents are primary metal oxides selected from iron oxides, titanium dioxide, titanium lower oxides, aluminum oxide, zirconuim oxides, cobalt oxides, cerium oxides, nickel oxides, or zink oxide, or composite oxides, more preferably an iron oxide selected from red iron oxide, yellow iron oxide or black iron oxide, or a mixture thereof. The total microencapsulated colorants are present in amounts ranging from 1% to 10%, preferably 3% to 7%; the sunscreen agents are present in amounts ranging from 5% to 50%, preferably 10% to 40%; the moistening agents are present in amounts ranging from 0.5% to 5%, preferably 1% to 3%; and the antioxidants are present in amounts ranging from 0.1% to 1%, preferably 0.2% to 0.5% by weight of the total composition.

In a more preferred embodiment, the multifunctional color-changing sunscreen/sunblock composition of the invention is a multifunctional high SPF (45) sunscreen/sunblock composition comprising the three primary iron oxide colorants yellow, red and black iron oxides, separately microencapsulated, non-encapsulated sunscreen agents titanium dioxide, zinc oxide, and homosalate, microencapsulated antioxidant vitamin E and moistening and calming agents Borage oil and allantoin.

In another preferred embodiment, the present invention provides a multifunctional color-changing after-sun/after tanning composition, which provides an immediate color indicative effect along with cooling, calming, and anesthetic actions. After-sun products should provide a relief in pain and discomfort caused by sunburn and compensate for the skin moisture loss. It is a common practice to include in after-sun compositions topical anesthetics that deaden the nerve edgings in the skin and relieve the pain, moistening actives that repair the skin's moisture balance, and cooling and calming active ingredients that provide a pleasant cool sensation to the burned skin areas. The multifunctional color-changing after-sun/after tanning composition provide, in addition to the color indicative effect a concealing effect and a healthy skin tone look to reddened areas of the sunburned skin.

In accordance with this specific embodiment, multifunctional after-sun lotion compositions comprise one or more, preferably three colorants, more preferably three colorants separately microencapsulated, and one or more active agents in a microencapsulated or non-capsulated form selected from a moistening, revitalizing, cooling, calming anesthetic agents or antioxidants/free radical scavengers. The microencapsulated colorants are present in amounts ranging from 2% to 8%, preferably 3% to 5%; the moistening agents are present in amounts ranging from 0.5% to 5%, preferably 1% to 3%; the revitalizing agents are present in amounts ranging from 2% to 10%, preferably 3% to 8%; the cooling agent is present in amounts ranging from 0.02% to 0.2%, preferably 0.05% to 0.15%; and the antioxidant is present in amounts ranging from 0.1% to 0.5%, preferably 0.2% to 0.3%, by weight of the total composition.

In a more preferred embodiment, the after-sun composition of the invention is a multifunctional after sun lotion comprising the three primary iron oxide colorants, yellow, red and black, separately microencapsulated, microencapsulated revitalizing agent Hippophae oil, microencapsulated antioxidants vitamin E and Grape Seed extract and microencapsulated cooling/calming agent menthol.

In a further preferred embodiment, the present invention provides a multifunctional color-changing sunless tanning composition, which provides an immediate "summer" skin tone. The common purpose of sunless tanning compositions is to provide a desired color shade of a tan tone to the skin, moisturize the skin and protect it from sunburn. The tan effect of known compositions is actually observed only a certain amount of time after application of the product to the skin. The color-changing sunless tanning compositions of the present invention provide an immediate desired tan skin tone to the face and body that accentuate the simultaneous delivery of other active ingredients beneficial to the skin, such as moistening and UV absorbers. When the sunless tanning composition contains a sunscreen agent, the colored areas of the skin indicate that those applied skin areas are protected from UV light.

In accordance with this specific embodiment, a multifunctional color-changing sunless tanning composition comprises one or more, preferably three colorants, more preferably three colorants separately microencapsulated, and one or more active agents in a microencapsulated or non-capsulated form selected from a sunless tanning agent, moistening agent, an antioxidant agent, or a sunscreen agents. The sunless tanning agent is present in amounts ranging from 2% to 8%, preferably 3% to 5%; the total microencapsulated colorants are present in amounts ranging from 2% to 8%, preferably 5% to 7%; the moistening agent is present in amounts ranging from 0.5% to 5%, preferably 1% to 3%; the antioxidant agent/free radical scavenger is present in amounts ranging from 0.1% to 0.5%, preferably 0.2% to 0.3%; and the sunscreen agent is present in amounts ranging from 2% to 6%, preferably 3% to 5%, by weight of the total composition.

In a more preferred embodiment, the multifunctional color-changing sunless tan composition of the invention is a sunless tan body lotion comprising the three primary iron oxide colorants, yellow, red and black, separately microencapsulated, non-encapsulated sunless tanning agent 1,3-dihydroxyacetone (DHA), and microencapsulated antioxidant vitamin E, moistening agents Borage oil and vitamin F, and the sunscreen agent octyl methoxycinnamate.

In another preferred embodiment, the present invention provides a multifunctional anti-aging skin care, which provides the color indication for delivery of the active ingredients as well as a make-up action. Anti-aging skin care products are aimed at preserving the beauty of the skin, which mainly corresponds to a young look and firm skin with even skin tone for prolonged periods of time. These products claim the benefits of a multitude of different activities on the skin such as protection, regeneration, moistening maintaining the skin in a balanced state or improving impaired skin conditions. The in situ development of a color shade immediately upon application of the anti-aging composition of the invention provides a desired healthy and even skin tone without an additional use of make-up.

In accordance with this specific embodiment, the color-changing anti-aging composition comprises one or more, preferably three colorants, more preferably three colorants separately microencapsulated, and one or more microencapsulated or non-encapsulated moistening, revitalizing/regenerating, antioxidant and sunscreen agents. The total micro-encapsulated colorants are present in amounts ranging from 1% to 6%, preferably 2% to 5%; the moistening agent is present in amounts ranging from 2% to 10%, preferably 4% to 7%; the revitalizing agent is present in amounts ranging from 0.1% to 2%, preferably 0.25% to 1%; the antioxidant is present in amounts ranging from 0.1% to 0.5%, preferably 0.2% to 0.3%; and the sunscreen agent is present in amounts ranging from 2% to 10%, preferably 5% to 7%, by weight of the total composition.

In a more preferred embodiment, the color-changing anti-aging skin care composition is a multifunctional facial cream comprising three primary iron oxide colorants, yellow, red and black, separately microencapsulated, microencapsulated moistening agent Evening Primrose Oil, microencapsulated vitamin A, microencapsulated antioxidant, non-encapsulated moistening agent allantoin, and the sunscreen agent avobenzone.

In another preferred embodiment, the color-changing composition is a multifunctional facemask composition containing active ingredients that provide intensive moistening, regenerating and revitalizing effects to the skin. The face mask composition may comprise the microencapsulated organic colorant D&C Green 6 and/or the three iron oxide pigments, the microencapsulated rejuvenating agent retinol, the antioxidant vitamin E, the astringent agent Witch Hazel distillate, the revitalizing/regenerating agent Panax Ginseng Root extract and the moistening agent vitamin F.

In still another preferred embodiment, the present invention provides a multifunctional color-changing whitening/lightening skin care composition, which provides the color indication for the delivery of the whitening active ingredients to the skin, and further provides a make-up action. Excessive production and accumulation of melanin in the skin related to prolonged exposure to the sun, may cause hyper pigmentation manifested in dark, irregular skin color and age spots on the face, back, arms, hands, and legs. Skin whitening/lightening agents lighten the skin by inhibiting tyrosinase activity and melanin synthesis. In general, the skin whitening products are applicable at night-time only. Otherwise, a full UV light protection is required due to the ability of the whitening agents to photosensitize the skin. The multifunctional whitening compositions of the present invention can be applied during day-time and outdoor activities due to the UV light protection provided by sunscreens and antioxidants, and masking effects on the pigmented skin area, and moreover, due to the indicative color effect which indicates the delivery of the active agents to the applied skin area.

In accordance with this specific embodiment, the color-changing whitening composition comprises one or more, preferably three colorants, more preferably three separately microencapsulated colorants, microencapsulated whitening/lightening agent and moistening, revitalizing, antioxidant, and sunscreen agents in microencapsulated or non-encapsulated form. The total microencapsulated colorants are present in amounts ranging from 1% to 10%, preferably 4% to 7%; the microencapsulated whitening/lightening agent is present in amounts ranging from 0.05% to 0.5%, preferably 0.1% to 0.3%; microencapsulated free radical scavenger is present in amounts ranging from 0.03% to 0.5%, preferably 0.06% to 0.3; and the sunscreen agent is present in amounts ranging from 2% to 20%, preferably 5% to 12%.

In a more preferred embodiment, the color-changing whitening/lightening composition is a multifunctional facial whitening cream comprising three primary iron oxide colorants, yellow, red and black, separately microencapsulated, microencapsulated whitening agent Licorice Root extract, microencapsulated antioxidant Grape Seed extract or vitamin E, and non-encapsulated sunscreen agent Solaveil CT-200.

In yet another preferred embodiment, the present invention provides a multifunctional color-changing aftershave composition, which possesses, a color indicating effect, a tonal/concealing effect, as well as a moistening, regenerating, calming, cooling, and an anti-inflammatory effects. Thus, the multifunctional aftershave of the invention provides a desired healthy looking skin, ameliorates skin's itching and reddening, and supports the healing of micro cuts and scars.

In a preferred embodiment, the color-changing aftershave composition comprises one or more, preferably three colorants, more preferably three separately microencapsulated colorants and the moistening, revitalizing/regenerating, cooling/calming, and anti-inflammatory agents in microencapsulated or non-capsulated forms. The microencapsulated colorants are present in amounts ranging from 0.5% to 5%, preferably 1% to 3%; the microencapsulated moistening, revitalizing, nourishing and regenerating agents are present in amounts ranging from 0.2% to 3%, preferably 0.5% to 1%; the microencapsulated calming/cooling agent is present in amounts ranging from 0.02% to 0.2%, preferably 0.05% to 0.1%; the microencapsulated anti-inflammatory agent is present in amounts ranging from 0.2% to 1%, preferably 0.5% to 0.8%, by weight of the total composition.

In a more preferred embodiment, the color-changing aftershave composition is a multifunctional calming aftershave balm, comprising three primary iron oxide colorants, yellow, red and black, separately microencapsulated, and microencapsulated moistening agent Borage oil, revitalizing/regenerating agent Hippophae oil, cooling/calming agent menthol, and the anti-inflammatory agent Tea Tree oil.

In yet still another preferred embodiment, the present invention provides a decorative multifunctional color-changing composition, more preferably a multifunctional make-up, that provides a desired even skin color coupled with moistening, sunscreen, and antioxidant activities. In accordance with this specific embodiment, the decorative composition comprises at least three separately microencapsulated colorants, moistening, antioxidant, and sunscreen agents in microencapsulated or non-capsulated forms. The microencapsulated colorants are present in amounts ranging from 2% to 10%, preferably 3% to 8%; the moistening agent is present in amounts ranging from 2% to 8%, preferably 4% to 7%; the antioxidant is present in amounts ranging 0.01% to 0.1%, preferably 0.01% to 0.05%; the sunscreen agent is present in amounts ranging from 2% to 10%, preferably 5% to 7%, by weight of the total composition.

In a further preferred embodiment, the present invention relates to a multifunctional color-changing dermal pharmaceutical composition comprising a blend of one or more, preferably three colorant, more preferably three separately microencapsulated colorants, wherein each colorant is a primary colorant. Such compositions provide the color indication for delivery and release of the therapeutic actives, and optionally further provide tonal/concealing effects, e.g. by masking skin disorders such as redness. The pharmaceutical compositions may comprise microencapsulated as well as non-capsulated antibiotic, antiviral, antifungal, general antiseptic, local anesthetic, keratolytic, steroid, non-steroid anti-inflammatory and antihistaminic agents for treatment of fever blisters (cold sores), eczema, psoriasis, skin blemishes, acne, shingles, pimples, cellulite, varicose veins, spider veins, rosacea and for strengthening capillary veins. The multifunctional color-changing pharmaceutical compositions may further provide protection from UV radiation without the need to apply additional sunscreen/sunblock products, and confer a healthy look to the skin immediately upon application. Such dermal products having both therapeutic and decorative actions may encourage teenagers to use them.

According to this specific embodiment, the multifunctional color-changing topical pharmaceutical composition comprises one or more separately microencapsulated colorants, microencapsulated or non-capsulated therapeutic agents e.g. antibiotics, and further microencapsulated or non-capsulated active ingredients selected from moistening agent, calming agent, antioxidant, and sunscreen agents. The microencapsulated colorants are present in amounts ranging from 3% to 10%, preferably 5% to 8%; antibiotic is present in amounts ranging from 0.05% to 0.2 %, preferably 0.1% to 0.15%; moistening agent is present in amounts ranging from 1% to 5%, preferably 2% to 3%; antioxidant/radical scavenger is present in amounts ranging from 0.1% to 0.5%, preferably 0.2% to 0.5%; the sunscreen agent is present in amounts ranging from 2% to 10%, preferably 5% to 7%; and the calming agent is present in amounts ranging from 0.2% to 1%, preferably 0.4% to 0.7%, by weight of the total composition.

In a more preferred embodiment, the present invention provides a color-changing multifunctional anti-acne face cream comprising one or more microencapsulated colorant, preferably a green colorant that provides a concealing effect and, optionally, the three primary iron oxide colorants, yellow, red and black, separately microencapsulated, and further comprising the anti-acne therapeutic agent salicylic acid (non-encapsulated), microencapsulated azithromycin, keratolytic agent vitamin A, antioxidant vitamin E, and cooling/calming agent menthol, non-encapsulated sunscreen agent benzophenone-3, and moistening agent Aloe Vera gel.

In another preferred embodiment, the present invention provides a multifunctional color-changing anti-cellulite composition with a color indicative effect for the delivery and release of the therapeutic Rutin hydrate (vitamin P), which further provides a slight concealing effect. In a more preferred embodiment, the composition comprises two or more, preferably three colorants, more preferably three separately microencapsulated colorants, microencapsulated Rutin hydrate, and moistening and antioxidant agents in microencapsulated or non-encapsulated form. Other actives beneficial for the skin such as sunscreen, astringent and revitalizing agents may also be incorporated. The microencapsulated colorants are present in amounts ranging from 3% to 8%, preferably 4% to 7%; the Rutin hydrate is present in amounts ranging from 0.05% to 0.3 %, preferably 0.1% to 0.2%; the moistening agent is present in amounts ranging from 1% to 5%, preferably 2% to 3%; the antioxidant/free radical scavenger is present in amounts ranging from 0.1% to 0.5%, preferably 0.2% to 0.5%, by weight of the total composition.

In a most preferred embodiment, the color-changing composition above is a multifunctional anti-cellulite cream comprising the three primary iron oxide colorants, yellow, red and black, separately microencapsulated, and microencapsulated Rutin hydrate, antioxidant Green Tea extract, keratolytic allantoin, astringent Witch Hazel, revitalizing Panax Ginseng Root extract and moistening agent vitamin F.

The invention will now be illustrated by the following non-limiting examples.

### EXAMPLES

The concentrations of the active substances either microencapsulated or not, are calculated as percentage by weight of whole composition. the concentration of the microencapsulated colorant are calculated as percentage by weight of whole composition of the loaded microcapsules.

**Materials.** The following microencapsulated materials were obtained from Tagra Biotechnologies Ltd., Netanya, Israel: yellow iron oxide (YellowCap1); red iron oxide (RedCapl); black iron oxide (BlackCap1); Ferric Ammonium Ferrocyanide (BlueCap); Retinol Palmitate - Vitamin A (Tagravit A2); Retinol (Tagravit R); free alpha-Tocopherol - Vitamin E (Tagravit E1); odorless mixture of free form of Linoleic and Linolenic acids (Tagravit F1); micro-capped masked Chamomile Oil (Tagrol CM1); Micro-capped masked Ginger Oil.(Tagrol GN1); Micro-capped masked Eucalyptus Citriodora Oil (Tagrol EC1); odorless Evening Primrose Oil (Tagrol EPO1); Borage Oil (Tagrol B1); Hippophae/Sea Buckthorn Oil (Tagrol H1); Tea Tree Oil (Tagrol TTO1); menthol (Tagrol Ment1); Microencapsulated Grape Seed Extract (Tagranat GS1); Microencapsulated Rutin Hydrate, (Tagranat Rutin1); Licorice (Tagranat Licorice1). These microencapsulated active agents are prepared as described in US Publication No. 2006/0051425 and are commercially available from Tagra Biotechnologies Ltd.

### Example 1. Preparation of composite double-layered microcapsules of iron oxides

First, the inner cores containing Iron Oxide pigments coated with a polymer-plasticizer shell were prepared by dissolving 2 gr Eudragit RS PO (Degussa) in 15 ml ethyl acetate while stirring for a period of 10 min. Then, 2 gr of a plasticizer chosen from tricaprylin, triethyl citrate, acetyl triethyl citrate, isopropyl myristate or a mixture thereof, was added while stirring for 5 min, followed by addition of 4 gr of an iron oxide pigments (chosen from Yellow Iron Oxide/hydroxide, Black Iron Oxide, Red Iron Oxide or a mixture thereof), while stirring for a period of 15 min. The obtained suspension was emulsified in 90 ml of water containing 0.5 gr PVA saturated beforehand with 15 ml ethyl acetate. This suspension was poured into 900 ml of water, while stirring, and incubated for 10-15 min to extract ethyl acetate and allow the formation of microcapsules. The obtained core microcapsules were isolated by sedimentation, washed with water and dried at a temperature not higher than 30°C to get a free flowing powder.

In the second stage, 6 gr of the inner core microcapsules were powdered with 0.01gr dioxosilicon (Aerosil 200, Degussa AG), in order to modify their outer surface.

In the third stage, a polymer-mineral dispersion was prepared by dissolving 1 gr Eudragit RS PO in 15 ml ethyl acetate while stirring for a period of 5 - 10 min. Then, 2 gr of a plasticizer chosen from tricaprylin, triethyl citrate, isopropyl myristate or a mixture thereof were added, while stirring for 5 min, followed by addition of 11 gr of a mineral chosen from titanium dioxide in the form of anatase, rutile, brookite, α-modification of boron nitride, magnesium silicate, potassium, sodium, magnesium hydroalumosilicate, magnesium myristate or mixture thereof. The preferred mineral used was usually boron nitride ("white graphite"), a soft solid lubricant that has flat graphitic hexagonal structure (h-BN, α-BN, or g-BN modification) with a particle size (D50) less than 10 µm.

The obtained dispersion was treated with ultrasonic for a period of 3 min. Then, 6 gr of the powdered modified inner cores microcapsules were gradually added to the dispersion while stirring for 5 min. After a homogenous suspension was obtained, it was emulsified in 84 ml of water containing 0.5 g PVA, saturated beforehand with 11 ml ethyl acetate. The obtained suspension was poured into 840 ml of water, while stirring, and incubated for a period of 3-5 min to extract ethyl acetate and allow formation of double-layered microcapsules. The isolation of the composite double-layered microcapsules as a free flowing powder was performed as described in the first stage. The outer diameter of the double-layer microcapsules thus obtained was in the range of 40-60 µm. Microscopic analysis revealed that the composite double-layered microcapsules are white spherical particles with a smooth surface. This result indicates that the outer shell completely masked the color of the incorporated pigments.

### Example 2. Preparation of high SPF multifunctional sunscreen composition

A multifunctional high SPF (sun protection factor) sunscreen composition containing a combination of inorganic and organic sunscreen agents, which produce an SPF 45 for skin protection against UVB/UVA, was prepared using the ingredients listed in Table 1, as follows: the ingredients of phase A were mixed in the order listed and heated to 70°C. Ingredients of phase B were combined and heated to 70°C. Phase A was added to Phase B slowly while mixing, and the mixing was continued for a further five minutes maintaining the temperature at 70°C. The mixture was then slowly cooled down to 45°C while mixing. Phase C (Fragrance) was added at 45°C. When the temperature decreased to 40°C the ingredients of phase D were added one by one, while mixing slowly, until uniform formulation was achieved. The ingredients of phase E were added at 40°C one at a time, whilst mixing gently, until a complete distribution is achieved. The composition was slowly cooled down to room temperature, under gentle mixing.

**Table 1. Ingredients for a 45 SPF sunscreen composition**

| **Phase** | **No** | **Ingredients** | **Commercial Name** | **% Weight** |
|---|---|---|---|---|
| **A** | 1 | Water | Water | Up to 100 |
| | 2 | Sodium chloride | Sodium Chloride | 0.56 |
| | 3 | Allantoin | Allantoin | 0.2 |
| **B** | 4 | Methylparaben | Nipagin M | 0.15 |
| | 5 | C12-C15 alkyl benzoate, titanium dioxide, Alumina, Methicone and polyhydroxystearic acid | TNP50T7 | 31.9 |
| | 6 | C12-C15 alkyl benzoate, zinc oxide, polyhydroxystearic acid and tri ethoxycaprylsil ane | TNP50ZSI | 10.0 |
| | 7 | Cetyl dimethicone | Abil Wax 9801 | 3.0 |
| | 8 | Homosalate | Kemester HMS | 11.0 |
| | 9 | Jojoba Esters | Floraesters 15 | 3.0 |
| | 10 | Shea Butter | Cetiol SB45 | 1.0 |
| | 11 | PEG-30 dipolyhydroxystearate | Arlacel P135 | 2.5 |
| | 12 | Polyglyceryl-4-Isosterate, cetyl dimethicone copolyol and hexyl laurate | Abil WE 09 | 2.5 |
| | 13 | Propylparaben | Nipagin P | 0.06 |
| | 14 | Octyl Palmitate | Crodamol OP | 3.0 |
| **C** | 15 | Fragrance | Fragrance | 0.5 |
| **D** | 16 | Microencapsulated vitamin E | Tagravit E1 | 1.0 |
| | 17 | Microencapsulated Borage oil | Tagrol B1 | 1.0 |
| **E** | 18 | Microencapsulated yellow iron oxide | YellowCap1 | 3.1 |
| | 19 | Microencapsulated red iron oxide | RedCap1 | 1.3 |
| | 20 | Microencapsulated black iron oxide | BlackCap1 | 0.6 |

The sunscreen cream obtained had a white color and when gently rubbed on the skin, immediately changed in color to a light tan tone.

### Example 3. Preparation of color-changing after sun lotion

A multifunctional after sun lotion composition which provides cooling, moistening, healing and light anesthetic effects to sunburned skin was prepared from the ingredients are listed in Table 2, as follows: the ingredients of phase A were combined in the listed order and heated to 75°C. The ingredients of phase B were combined and heated to 80°C. Phase B was slowly added to phase A, while homogenizing. Homogenization was continued for a further 5 minutes at a temperature of 75-80°C. The mixture was then slowly cooled down to 45°C with constant mixing, and the phase C (Fragrance) was added. The ingredients of phase D were added one at time under constant mixing at 40°C. The ingredients of phase E were premixed slightly at room temperature and slowly added to the mixture at 40°C, whilst mixing gently until complete distribution was achieved. The microencapsulated colorants of phase F were added gradually and slowly, one by one while mixing gently, until a uniform composition was obtained. The composition was slowly cooled down to room temperature, while gently mixing.

**Table 2. Ingredients for after sun lotion**

| **Phase** | **No** | **Ingredients** | **Commercial Name** | **% Weight** |
|---|---|---|---|---|
| | 1 | Water | Water | Up to 100 |
| | 2 | Glycerin | Glycerin | 3.0 |
| **A** | 3 | Cetearyl alcohol and sodium cetearyl sulfate | Lanette N | 2.0 |
| | 4 | Propylene glycol | Propylene Glycol | 3.0 |
| | 5 | Methylparaben | Nipagin M | 0.3 |
| | 6 | Chlorophenesin | Chlorophenesin | 0.2 |
| | 7 | Isohexadecane | Arlamol HD | 1.0 |
| | 8 | Dimethicone | Dow 350 | 0.8 |
| **B** | 9 | Cetyl alcohol | Lanette 16 | 2.0 |
| | 10 | Cetearyl alcohol and PEG-20 stearate | Polawax GP200 | 3.0 |
| | 11 | Octyl palmitate | Crodamol OP | 5.0 |
| | 12 | Propylparaben | Nipagin P | 0.2 |
| **C** | 13 | Fragrance | Fragrance | 0.5 |
| **D** | 14 | Aloe Barbadensis gel | Aloe Vera Gel x 10 | 1.0 |
| | 15 | Witch Hazel (Hamamelis Virginiana) Distillate | Witch Hazel Distillate | 1.0 |
| **E** | 16 | Microencapsulated menthol | Tagrol Ment1 | 1.0 |
| | 17 | Microencapsulated vitamin E | Tagravit E1 | 1.0 |
| | 18 | Microencapsulated Grape (Vitis Vinifera) Seed extract | Tagranat GS1 | 1.0 |
| | 19 | Microencapsulated Hippophae (Sea Buckthorn) oil | Tagrol H1 | 1.0 |
| | 20 | Aluminum starch octenyl succinate | Dry Flo PC | 1.0 |
| **F** | 21 | Microencapsulated yellow iron oxide | YellowCap1 | 2.1 |
| | 22 | Microencapsulated red iron oxide | RedCap1 | 0.6 |
| | 23 | Microencapsulated black iron oxide | BlackCap1 | 0.3 |

### Example 4. Preparation of a color-changing sunless tanning lotion

A multifunctional color-changing sunless tanning body lotion containing sunless tanning agent and active ingredients for moistening, protecting and revitalizing the skin was prepared using the ingredients listed in Table 3, as follows: the ingredients of phase A were combined in the order listed and heated to 75°C. The ingredients of phase B were combined and heated to 80°C. Phase B was added slowly to phase A, while homogenizing. Homogenization was continued for a further 5 minutes at a temperature of 75-80°C. The mixture was slowly cooled down to 45°C with mixing, and then phase C (Fragrance) was added. The ingredients of phase D were combined and warmed slowly up to a maximum of 35-40°C with constant stirring until the phase was cleared. The prepared phase D was added to the mixture at the temperature of 35-40°C, while mixing. The ingredients of phase E were added slowly, one at a time, to the emulsion at 40°C, whilst gently mixing until complete distribution was achieved. The ingredients of phase F were then added gradually, one at a time, while gently mixing at 40°C until uniform composition was obtained. The composition was slowly cooled down to room temperature, while gently mixing.

The color-changing sunless tanning body lotion obtained had a white color and when gently rubbed on the skin, immediately changed its color and provided a bronze tan tone to the applied skin area.

**Table 3. Ingredients for sunless tanning body lotion**

| **Phase** | **No** | **Ingredients** | **Commercial Name** | **% Weight** |
|---|---|---|---|---|
| **A** | 1 | Water | Water | Up to 100 |
| | 2 | Glycerin | Glycerin | 3.0 |
| | 3 | Cetearyl alcohol and sodium cetearyl | Lanette N | 2.0 |
| | 4 | Propylene glycol | Propylene Glycol | 3.0 |
| | 5 | Methylparaben | Nipagin M | 0.3 |
| | 6 | Imidazolidinyl urea | Germall 115 | 0.2 |
| **B** | 7 | Octyl methoxycinnamate | Escalol | 3.0 |
| | 8 | Dimethicone | Dow 350 | 0.8 |
| | 9 | Cetyl alcohol | Lanette 16 | 3.0 |
| | 10 | Cetearyl alcohol and PEG-20 stearate | Polawax GP200 | 3.0 |
| | 11 | Octyl palmitate | Crodamol OP | 5.0 |
| | 12 | Propylparaben | Nipagin P | 0.2 |
| **C** | 13 | Fragrance | Fragrance | 0.5 |
| **D** | 14 | Water | Water | 7.0 |
| | 15 | Dihydroxyacetone | DHA | 5.0 |
| **E** | 16 | Micrencapsulated Borage oil | Tagrol B1 | 1.0 |
| | 17 | Microencapsulated vitamin F | Tagravit F1 | 1.0 |
| | 17 | Microencapsulated vitamin E | Tagravit E1 | 1.0 |
| | 18 | Lauryl pyrrolidone | Sulfadone LP-300 | 2.0 |
| | 19 | Aluminum starch octenyl succinate | Dry Flo PC | 3.0 |
| **F** | 20 | Microencapsulated yellow iron oxide | YellowCap1 | 3.6 |
| | 21 | Microencapsulated red iron oxide | RedCap1 | 0.9 |
| | 22 | Microencapsulated black iron oxide | BlackCap1 | 0.5 |

### Example 5. Preparation of color-changing calming aftershave balm

A multifunction color-changing calming aftershave balm containing active ingredients capable of moistening, calming/cooling, protecting and revitalizing the skin was prepared using the ingredients listed in Table 4, as follows: the ingredients of phase A were combined in the order listed and heated to 80°C. The ingredients of phase B were combined and heated to 80°C. Then, phase A was slowly added to phase B, while homogenizing. The mixing was continued for further 15 minutes at the temperature 75-80°C, and the mixture was then slowly cooled down to 40°C under mixing. Phase C was prepared by slurring aluminium starch octenylsuccinate into glycerine and adding to the mixture of phases A and B at 40°C. Then phenoxyethanol was added and the resulting emulsion was mixed well, maintaining the same temperature at 40°C. Phase D (Fragrance) was added while mixing. The ingredients of phase E were added slowly, one at a time, under a gentle mixing at 40°C until a uniform composition was obtained. The composition was slowly cooled to room temperature, while gently mixing.

The aftershave balm obtained had a white color and when gently rubbed on the skin, immediately changed its color and provided an even, natural skin tone to the applied skin area.

**Table 4. Ingredients for the calming aftershave balm**

| **Phase** | **No** | **Ingredients** | **Commercial Name** | **% Weight** |
|---|---|---|---|---|
| **A** | 1 | n-Butyl stearate | Stepan BS | 4.0 |
| | 2 | Cetyl palmitate | Dermol CP | 2.5 |
| | 3 | Myristyl propionate | Schercemol MP | 3.0 |
| | 4 | Mineral oil and PEG-30 lanolin; Cetearyl alcohol | Base SE | 1.5 |
| | 5 | Propylparaben | Nipagin P | 0.15 |
| **B** | 6 | Water | Water | Up to 100 |
| | 7 | Lecitin | Lecitin | 1.0 |
| | 8 | Carbomer | Carbopol 5984 | 10.0 |
| | 9 | Sodium hydroxide | Sodium Hydroxide | 0.4 |
| | 10 | Methylparaben | Nipagin M | 0.15 |
| **C** | 11 | Glycerin | Glycerin | 7.0 |
| | 12 | Aluminum starch octenyl succinate | Dry Flo PC | 8.0 |
| | 13 | Phenoxyethanol | 2-Phenoxyethanol | 0.2 |
| **D** | 14 | Fragrance | Fragrance | 0.5 |
| **E** | 15 | Microencapsulated menthol | Tagrol Ment 1 | 1.0 |
| | 16 | Microencapsulated Borage oil | Tagrol B1 | 1.0 |
| | 17 | Microencapsulated Tea Tree oil | | |
| | 18 | Microencapsulated Hippophae (Sea Buckthorn) oil | Tagrol H1 | 2.0 |
| | 19 | Microencapsulated yellow iron oxide | YellowCap1 | 1.4 |
| | 20 | Microencapsulated red iron oxide | RedCap1 | 0.46 |
| | 21 | Microencapsulated black iron oxide | BlackCap1 | 0.24 |

### Example 6. Preparation of a color-changing whitening face cream

A multifunctional whitening face cream containing active ingredients capable of de-pigmenting or whitening/lightening sun, aging, and pregnancy pigmentation on the skin and providing extremely strong antioxidant/free radical scavenger properties was prepared from the active ingredients listed in Table 5, as follows: the ingredients of phase A (with the exception of Solaveil CT 200 and DC 345 Fluid) were mixed and heated to 75-80°C. The sunscreen agent Solaveil CT 200 was then added to the mixture when all other ingredients were already melted. Phase B was prepared as follows: Alphanta was added to the water and the mixture was heated to 45-50°C, then glycerin and Xanthan Gum were mixed and added to the warm water, under stirring. The stirring was continued for a further 20-30 min, maintaining the same temperature and then the mixture was slowly heated up to 75-80°C. Phase A was added to phase B under moderate stirring, then DC 345 Fluid was added and the resulting mixture of phases A and B was homogenized for further 5 minutes. The obtained emulsion was cooled down to 40-45°C, while stirring slowly. The ingredients of phase C were combined and heated to 45°C, and then added to the mixture of phases A and B, and mixed for further 5 minutes. The Kemaben 2 (phase D) (mixture of Diazolidinyl Urea, Methylparaben, Propylparaben, Propylene Glycol) was added to the emulsion at 45°C under permanent mixing. The fragrance (phase E) was added at the same temperature and the mixing was continued for further 15 minutes, maintaining the temperature at 40-45°C until a uniform composition is was achieved. The pH of the composition was adjusted around 7.0 with a NaOH 20-25% solution. The ingredients of phase F were combined and slowly and gradually incorporated into the formulation while gently mixing, until complete distribution was achieved. The composition was slowly cooled down to room temperature while gently mixing.

The whitening/lightening face cream obtained had a white color. Upon gently rubbing the cream of a skin area covered with dark spots, the cream changed its color, concealed the spots and provided the applied akin area with an even tone.

**Table 5. Ingredients for whitening face cream**

| **Phase** | **No** | **Ingredients** | **Commercial Name** | **% Weight** |
|---|---|---|---|---|
| **A** | 1 | Glyceryl stearate PEG-100 stearate | Arlacell 165 F1 | 6.0 |
| | 2 | Polysorbate 60 | Tween 60 | 0.8 |
| | 3 | Dipolyhydroxystearic acid | Arlacell P135 | 0.5 |
| | 4 | Hydrogenated polyisobutene | Prisorine 3758 | 8.0 |
| | 5 | Titanium dioxide, triethylhexanoin, Isohexadecane, aluminum stearate, Alumina, polyhydroxystearic acid | Solaveil CT200 | 10.0 |
| | 6 | Kojic dipalmitate | KAD-15 | 2.0 |
| | 7 | Cetearyl alcohol | Ecorol 68/30 | 1.5 |
| | 8 | Cyclopentasiloxane | CD345 Fluid | 5.0 |
| | 9 | Dimethicone | DC 100/100 | 1.0 |
| **B** | 10 | Water | Water | Up to 100 |
| | 11 | Panthenol allantoin | Alpantha | 0.1 |
| | 12 | Glycerin | Pricerine 9091 | 3.0 |
| | 13 | Xanthan gum | Rhodicare S | 0.2 |
| **C** | 14 | Water | Water | 5.0 |
| | 15 | Magnesium ascorbyl phosphate | MAP-SL | 1.0 |
| **D** | 16 | Propylene glycol, diazolidinyl urea, methylparaben and propylparaben | Kemaben 2 | 1.0 |
| **E** | 17 | Fragrance | Fragrance | 0.5 |
| **F** | 18 | Micrencapsulated licorice (Glycyrrhiza Glabra) Root extract | Tagranat Licorice 1 | 1.25 |
| | 19 | Microencapsulated Grape (Vitis Vinifera) Seed extract | Tagranat GS1 | 1.0 |
| | 20 | Microencapsulated yellow iron oxide | YellowCap1 | 3.0 |
| | 21 | Microencapsulated DC Red 7 | Red7Cap1 | 0.7 |
| | 22 | Microencapsulated black iron oxide | BlackCap 1 | 0.3 |

### Example 7. Preparation of a color-changing anti-acne face cream

A multifunctional color-changing anti-acne cream containing active ingredients which control the acne breakouts was prepared from the ingredients listed in Table 6, as follows: ingredients of phase A were combined and mixed for 15 minutes, while heating to 70-75°C. Ingredients of phase B were combined and heated to 75-80°C. After both phases reached their respective temperatures, phase B was added to phase A while homogenizing. Homogenization was continued for further 15 minutes, and then the emulsion was cooled down to 40-45°C. The components of phase C were combined and added to the mixture of phases A and B at a temperature of around 40°C. The ingredients of phase D were combined at room temperature and stirred until a complete dissolution of salicylic acid was achieved. Phase D was added to the mixture of phases A, B and C at room temperature under gentle mixing. Phase E was premixed and added to the rest formulation at room temperature. The formulation was mixed for further 5-10 minutes until a substantially uniform composition was achieved. The microencapsulated ingredients of phase F were added gradually, one at a time, and the composition was mixed gently until a complete distribution of the ingredients was obtained.

The anti-acne cream obtained, immediately changed its color to a natural to slight tanned skin tone upon gentle rubbing on the skin, and effectively and evenly concealed damaged skin area.

**Table 6. Ingredients for anti-acne face cream**

| **Phase** | **No** | **Ingredients** | **Commercial Name** | **% Weight** |
|---|---|---|---|---|
| **A** | 1 | Water | Water | Up to 100 |
| | 2 | Glycerin | Pricerine 9088 | 3.5 |
| | 3 | Hydroxypropyl starch phosphate | Structure XL | 4.0 |
| | 4 | Aloe vera gel | | 2.0 |
| **B** | 5 | PPG-11 stearyl ether | VARONIC APS | 9.0 |
| | 6 | Cetearyl alcohol | Lannete Wax O | 4.0 |
| | 7 | Cyclomethicone and dimethiconol | Dow Coming 1401 | 0.8 |
| | 8 | Dimethicone | Mirasil DM | 1.2 |
| | 9 | Steareth-2 | Brij 72 | 1.05 |
| | 10 | Steareth-21 | Brij 721 | 2.45 |
| | 11 | Benzophenone-3 | Escalol 567 | 5.0 |
| **C** | 12 | Propylene glycol, diazolidinyl urea, methylparaben and propylparaben | Paragon G2 | 1.0 |
| | 13 | Tetrasodium EDTA | Versene 100 | 0.29 |
| **D** | 14 | Glycereth-7 trimethyl ether | Coscap G7-C | 5.0 |
| | 15 | Salicylic acid | Salicylic Acid | 2.0 |
| **E** | 16 | Water | Water | 0.5 |
| | 17 | Triethanolamine | Triethanolamine | 0.2 |
| **F** | 18 | Microencapsulated azithromycin | | 0.5 |
| | 19 | Microencapsulated vitamin A | Tagravit A | 1.0 |
| | 20 | Microencapsulated vitamin E | Tagravit E | 1.0 |
| | 21 | Microencapsulated menthol | Tagrol Menth | 1.0 |
| | 22 | Microencapsulated chromium oxide green | GreenCap1 | 5 |

### Example 8. Preparation of a color-changing anti-cellulite body cream

A multifunctional color-changing anti-cellulite body cream containing active ingredients capable of improving the skin's texture and appearance was prepared from the ingredients listed in Table 7, as follows: the ingredients of phase A were combined and heated to 75°C. The ingredients of phase B were combined and heated to 80°C. Phase B was slowly added to phase A whilst homogenizing, which was continued for further 5 minutes at a temperature of 75-80°C. The emulsion was slowly cooled down to 50°C while mixing. The fragrance (phase C) was added at 50°C during mixing. The mixture was cooled, and the ingredients of phase D were added one at time at 40°C with vigorous mixing. The mixing was continued until uniform consistency was achieved. The ingredients of phase E were added at 40°C gradually, one at a time, with gently and slowly mixing until a complete distribution was obtained. The composition was slowly cooled down with a gentle mixing.

The white anti-cellulite face cream obtained, changed its color upon gently rubbing it on a cellulite area, masked the cellulite signs and provided the applied area with an even and natural skin tone.

**Table 7. Ingredients for anti-cellulite body cream**

| **Phase** | **No** | **Ingredients** | **Commercial Name** | **% Weight** |
|---|---|---|---|---|
| **A** | 1 | Water | Water | Up to 100 |
| | 2 | Glycerin | Glycerin | 3.0 |
| | 3 | PEG-40 stearate | Mirj 52 | 1.0 |
| | 4 | Propylene glycol | Propylene Glycol | 3.0 |
| | 5 | Methylparaben | Nipagin M | 0.3 |
| | 6 | Chlorophenesin | Chlorophenesin | 0.3 |
| | 7 | Allantoin | Allantoin | 0.1 |
| **B** | 8 | Isohexadecane | Arlamol HD | 6.0 |
| | 9 | Glyceryl stearate | Cutina GMS | 4.0 |
| | 10 | Cetyl alcohol | Lanette 16 | 3.0 |
| | 11 | Sorbitan tristearate | Span 65 | 1.0 |
| | 12 | Cyclomethicone | Dow 344 | 3.0 |
| | 13 | Propylparaben | Nipagin P | 0.2 |
| **C** | 14 | Fragrance | Fragrance | 0.5 |
| **D** | 15 | Panax Ginseng Root extract | Ginseng extract | 1.0 |
| | 16 | Witch Hazel (Hamamelis Virginiana) distillate | Witch Hazel Distillate | 3.0 |
| | 17 | Camellia Sinensis Leaf extract | Green Tea extract | 1.0 |
| **E** | 18 | Microencapsulated Rutin hydrate (vitamin P) | Tagranat Rutin1 | 2.0 |
| | 19 | Microencapsulated vitamin F | Tagravit F1 | 1.0 |
| | 20 | Microencapsulated yellow iron oxide | YellowCap1 | 3.0 |
| | 21 | Microencapsulated red iron oxide | RedCap1 | 0.9 |
| | 22 | Microencapsulated black iron oxide | BlackCap1 | 0.2 |

### Example 9. Preparation of a color-changing anti-aging face cream

A multifunctional anti-aging face cream composition containing active ingredients capable of moistening, renewing, regenerating, revitalizing and protecting the face skin from aging processes was prepared from the ingredients listed in Table 8, as follows: the components of phase A were combined and heated to 75°C. The components of phase B were combined and heated to 80°C. Phase B was added slowly to phase A, while homogenizing. Homogenization was continued for a further 5 minutes at 75-80°C. The mixture was then cooled to 60°C and phase C was added, while homogenizing for further 5 minutes at the same temperature (60°C). The components of phase D were added slowly while gently stirring. Stirring was continued until the complete dispersion of phase D in the carrier was achieved, and the resulting mixture was allowed to cool. The fragrance (phase E) was added at 45°C, while stirring. When the temperature had decreased to 40°C the ingredients of phase F were added one at a time. A slow and gentle mixing was continued until the formulation achieved substantial uniformity. The microencapsulated colorants of phase G were added gradually, one at a time, and slow mixing was continued until a complete distribution was achieved. The composition was cooled down to room temperature while gently mixing.

The anti-aging face cream obtained had a white color. Upon gently rubbing the cream of a skin area the cream changed its color, and provided the applied akin area with an even tone and natural skin tone.

**Table 8. Ingredients for anti-aging face cream**

| **Phase** | **No** | **Ingredients** | **Commercial Name** | **% Weight** |
|---|---|---|---|---|
| **A** | 1 | Water | Water | Up to 100 |
| | 2 | Glycerin | Glycerin | 2.0 |
| | 3 | Allantoin | Allantoin | 0.1 |
| | 4 | Tetrasodium EDTA | Versene 100 | 0.1 |
| | 5 | Propylene glycol | Propylene Glycol | 3.0 |
| | 6 | Methylparaben | Nipagin M | 0.3 |
| | 7 | Xanthan gum | Keltrol T | 0.4 |
| **B** | 8 | Avobenzone | Eusolex 9020 | 6.0 |
| | 9 | Decyl oleate | Cetiol V | 1.5 |
| | 10 | Myristyl myristate | Cetiol MM | 1.0 |
| | 11 | Steareth-21 | Brij 721 | 2.4 |
| | 12 | Steareth-2 | Brij 72 | 3.6 |
| | 13 | PPG-15 stearyl ether | Arlamole E | 2.0 |
| | 14 | Cetyl alcohol | Lanette 16 | 1.5 |
| | 15 | Cyclomethicone and dimethiconol | Dow 1503 | 1.0 |
| | 16 | Octyl Palmitate | Crodamol OP | 3.0 |
| | 17 | Phenoxyethanol, methyparaben, butylparaben, Ethylparaben and Propylparaben | Phenonip | 0.6 |
| **C** | 18 | Polyacrylamide & C13-14 Isoparaffin&Laureth-7 | Sepigel 305 | 1.0 |
| **D** | 19 | Microencapsulated Evening Primrose oil | Tagrol EPO1 | 1.0 |
| | 20 | Microencapsulated vitamin A | Tagravit A1 | 1.0 |
| **E** | 21 | Fragrance | Fragrance | 0.4 |
| **F** | 22 | Chlorohexidine digluconate | Chlorohexidine Digluconate | 1.0 |
| | 23 | Ammonium lactate | Pursal NH70 | 0.6 |
| | 24 | Lactic acid | Lactic acid | 0.3 |
| **G** | 25 | Microencapsulated yellow iron oxide | YellowCap1 | 2.3 |
| | 26 | Microencapsulated red iron oxide | RedCap1 | 0.5 |
| | 27 | Microencapsulated black iron oxide | BlackGap1 | 0.2 |

### Example 10. Preparation of a color-changing facemask

A color-changing face mask containing active ingredients capable of providing intensive moistening, regenerating and revitalizing effects to the skin was prepared from the ingredients listed in Table 9, as follows: phases A and B were heated to 85°C separately. Then, Phase B was added to phase A and homogenized during 10-15 minutes at 80-85°C. The emulsion was cooled down to about 40°C and the ingredients of phases C and D were added one at a time, while homogenizing and maintaining the temperature at around 40°C. The ingredients of phase E were then added to the formulation gradually one at a time, at a temperature of around 40°C with gentle mixing until a complete distribution was obtained. The composition was cooled down to room temperature, with a gentle mixing until a uniform composition was obtained.

**Table 9. Ingredients for a facemask**

| **Phase** | **No** | **Ingredients** | **Commercial Name** | **% Weight** |
|---|---|---|---|---|
| **A** | 1 | Ceteareth-25 | Lipocol SC-25 | 2.0 |
| | 2 | Ceteareth-6 and stearyl alcohol | Cremophor A6 | 2.0 |
| | 3 | Glyceryl stearate | Lipo GMS-450 | 6.0 |
| | 4 | Cetyl alcohol | Lannete-16 | 1.0 |
| | 5 | Cetearyl alcohol | Ecorol 68/30f | 4.0 |
| | 6 | Cetearyl ethylhexanate | Dermol JOBA | 5.0 |
| **B** | 7 | Propylene glycol | Propylene Glycol | 5.0 |
| | 8 | Disodium EDTA | Disodium EDTA | 0.1 |
| | 9 | Panthenol | Panthenol | 3.0 |
| | 10 | Kaolin | Kaolin | 6.0 |
| | 11 | Phenoxyethanol, methyparaben, butylparaben, Ethylparaben and Propylparaben | Phenonip | 0.6 |
| | 12 | Water | Water | Up to 100 |
| **C** | 13 | Bisabolol | | 1.0 |
| | 14 | Fragrance | Fragrance | 0.3 |
| **D** | 15 | Witch Hazel (Hamamelis Virginiana) distillate | Witch Hazel Distillate | 3.0 |
| | 16 | Panax Ginseng Root extract | Ginseng extract | 2.0 |
| | 17 | Sodium ascorbyl phosphate | Ascorbyl PS | 1.0 |
| E | 18 | Microencapsulated Retinol | Tagravit R | 2.0 |
| | 19 | Microencapsulated vitamin E | Tagravit E | 2.0 |
| | 20 | Microencapsulated vitamin F | Tagravit F | 1.0 |
| | 21 | Microencapsulated Colorant D&C Green 6 Liposoluble | GreenCapl | 5.0 |

## Claims

1. A color-changing composition for skin care that provides a changing color effect that indicates delivery of one or more active substances onto the skin, the composition comprising
(i) one or more active substances for skin care selected from moistening, healing/regenerating/revitalizing, depigmentation/whitening/lightening, antioxidant/radical scavenger, cooling/calming, warming, anesthetic, sunscreen/sunblock, antibiotics, anti-cellulite, keratolytic, antifungal, antipsoriatic, anti-inflammatory, antibacterial, astringent, antiseptic, repellent, anti-spider vein, anti-rosacea/anti-couperosis, anti-acne agents, fragrances/scents or agents for treatment of fever blisters; and
(ii) one or more microencapsulated colorants;
wherein
said one or more microencapsulated colorants are encapsulated individually or as a blend of colorants in the core of double- or multi-layer microcapsules, said core containing the one or more colorants being surrounded by two or more shells of the same or different wall-forming polymer, and
said composition immediately and irreversibly changes its color upon application on the skin.

2. The color-changing composition according to claim 1, wherein the colorants are selected from organic pigments, inorganic pigments, lakes, natural and synthetic dyes or any combination thereof.

3. The color-changing composition according to claim 2, wherein said colorants are selected from metal oxides, preferably iron oxides, titanium dioxide, chromium oxide, zinc oxide and composite metal oxides; metal hydroxides such as calcium hydroxide, iron hydroxides, aluminum hydroxide, chromium hydroxide, magnesium hydroxide and composite metal hydroxides; other colorants such as ferric ammonium ferrocyanide, Prussian blue, iron sulfides, manganese violet, carbon black, mica, kaolin; Lake organic pigments such as Indigo Lakes, Carmine Lakes, lakes from the series FD&C and D&C dyes such as D&C Red 21 Aluminum Lake, D&C Red 7 Calcium Lake; or a natural or synthetic organic dye selected from aromatic azo, indigoid, triphenylmethane, anthraquinone or xanthine dyes of the D&C and FD&C series.

4. The color-changing composition according to any of claims 1 to 3, wherein the one or more active substances for skin care are in microencapsulated or in non-capsulated form, and upon application on the skin the composition provides a changing color effect which is an indicator of the delivery of the one or more active substances to the area of application on the skin.

5. The color-changing composition according to claim 1, wherein the one or more active substances for skin care are selected from:
(i) moistening or moisturizing agents such as, but not limited to, Evening Primrose oil, Borage oil, Jojoba oil, Aloe vera gel, Vitamin F, panthenol, and mixtures thereof;
(ii) healing/revitalizing/regenerating agents such as, but not limited to, Hippophae (Sea Buckthorn) oil, Tea Tree oil, allantoin and derivatives, carotenoids, Carrot Seed extract and oil, Patchouli essential oil, and mixtures thereof;
(iii) depigmentation (whitening)/lightening agents such as, but not limited to, Licorice (Gycyrrhiza Glabra) root extract, arbutin, kojic acid, hydroquinone, vitamin C and derivatives, and mixtures thereof;
(iv) antioxidant/free radical scavenging agents such as, but not limited to, vitamin E, tocotrienols, tocopherols, vitamin F, vitamin C and derivatives thereof, Rutin, Resveratrol and derivatives thereof, Camellia Sinensis leaf extract (Green Tea or Black Tea), Grape Seed extract, Evening Primrose oil, Borage oil, Ginger essential oil, curcumin, chitosan, and mixtures thereof;
(v) cooling and calming agents such as, but not limited to, menthol, Aloe Barbadensis leaf extract, camphor, menthyl lactate, allantoin, bisabalol, Chamomile extract and essential oil, Evening Primrose oil, Borage oil, Eucalyptus Citriodora essential oil, Patchouli essential oil, panthenol, and mixtures thereof;
(vi) warming agents such as, but not limited to, black pepper extract and essential oil, paprika (red pepper) extract, Cinnamon extract and essential oil, Ginger root extract and essential oil, zeolite; and mixtures thereof;
(vii) anesthetic agents such as, but not limited to, Aloe barbadensis gel (Aloe vera gel), benzocaine, lidocaine, dibucaine, pramoxine, tetracaine, camphor, resorcinol, and mixtures thereof;
(viii) sunscreen/sunblock agents such as, but not limited to, p-aminobenzoic acid (PABA) and esters thereof, benzalphthalides, benzophenones, cinnamates, etocrylene, octocrylene, Grape Seed extract, titanium dioxide, zinc oxide, and mixtures thereof;
(ix) antibiotics for topical application such as, but not limited to, erythromycin, clarithromycin, azithromycin and clindamycin;
(x) anti-cellulite agents such as, but not limited to, Rutin hydrate;
(xi) keratolytic agents useful for treating acne, warts and other skin diseases such as, but not limited to, benzoyl peroxide, sulfur, aminolevulinic acid, fluorouracil, podophyllotoxin, podophyllum, propylene glycol, phytic acid, and mixtures thereof;
(xii) antibacterial agents such as, but not limited to, antibiotics, Chamomile essential oil;
(xiii) antifungal agents such as, but not limited to, Tea Tree oil, Grape Seed extract, Eucalyptus Citriodora essential oil, ursolic acid, essential oils, amphotericin, itaconazole, fluconazole, ketoconazole, miconazole, morpholine, undecylenic acid, and mixtures thereof;
(xiv) anti-acne agents such as, but not limited to, benzoyl peroxide, Chamomile essential oil, and mixtures thereof;
(xv) antipsoriatic agents such as, but not limited to, vitamin C, vitamin D3 and analogs thereof, ergocalciferol, anthralin, metronidazole, tazarotene, cyclosporine, pyrogallol, allantoin, and mixtures thereof;
(xvi) anti-inflammatory agents such as, but not limited to, vitamin F, vitamin E, unsaturated fatty acids, Rutin, bioflavanoids, Hippophae (Sea Buckthorn) oil, Olive oil, Salicin, Ginger root extract and essential oil, Jojoba oil, Chamomile essential oil, Eucalyptus Citriodora essential oil, ursolic acid, triamcinolones, cortisones, prednisones, cortodoxone, flucetonide, medrysone, amcinafel, amcinafide, betamethasone and esters thereof, clocortelone, descinolone, desonide, flucloronide, flumethasone, flunisolide, fluocinonide, flucortolone, paramethasone, dexamethasone, fluroandrenolone acetonide, acetonide, dichlorisone, and mixtures thereof;
(xvii) astringent agents such as, but not limited to, Witch hazel (Hamamelis Virginiana), Yarrow (Achillea millefolium), Rosewood oil, benzoin, aluminum sulfate and other aluminum salts, zinc oxide, and mixtures thereof;
(xviii) antiseptic agents such as, but not limited to, honey, curcumin, captan, chlorhexidine and its derivatives, hexachlorophene, triclosan, triacetin, sodium usnate, sulfur, and mixtures thereof;
(xix) repellent agents such as, but not limited to, essential oils, Pyrethrin, Permethrin, Bioresmethin, dimethylphthalate, and mixtures thereof;
(xx) antirosacea agents such as, but not limited to, Vitamin K, sulfur, Marigold oil, Rutin, bioflavonoids, and mixtures thereof;
(xxi) active substances beneficial for fever blisters (cold sores and shingles) local treatment including, but not limited to, acyclovir, antihistamines, local anesthetic, essential oils possessing antiviral activity, and mixtures thereof; and
(xxii) fragrances/scents including, but not limited to, lavender, Neroli fragrance oil, Chamomile essential oil, Ginger essential oil, Patchouli essential oil, Eucalyptus Citriodora essential oil, Rosemary essential oil, Sandalwood essential oil, Tea Tree oil, and mixtures thereof.

6. The color-changing composition according to any one of claims 1 to 5, formulated as an oil-in-water, oil-in-water-in-oil, water-in-oil, or a water-in-oil-in water emulsion, an aqueous formulation or an anhydrous formulation.

7. The color-changing cosmetic or therapeutic composition according claim 6, wherein said composition is a multifunctional composition selected from body skin care, facial skin care, baby care, sunscreen care, after sun, whitening, aftershave, anti-acne, anti-repellent, anti cellulite and anti-perspirant compositions.

8. The multifunctional color-changing cosmetic composition according to claim 7 selected from:
(i) a sunscreen/sun-block composition comprising one or more, preferably three or more colorants selected from iron oxides, titanium dioxide, titanium lower oxides, aluminum oxide, zirconuim oxides, cobalt oxides, cerium oxides, nickel oxides, or zink oxide, or composite oxides, preferably an iron oxide selected from red iron oxide, yellow iron oxide or black iron oxide, or a mixture thereof, preferably separately microencapsulated, and one or more microencapsulated or non-capsulated active agents selected from sunscreen agents preferably both organic and inorganic sunscreen agents, antioxidant/radical scavenger and moistening agents;
(ii) an after-sun/after tanning composition comprising one or more, preferably three colorants, more preferably the three primary iron oxide colorants, yellow, red and black, separately microencapsulated, and one or more active agents in a microencapsulated or non-capsulated form selected from moistening, revitalizing, cooling, calming anesthetic agents or antioxidants/free radical scavengers;
(iii) an anti-aging facial cream comprising one or more, preferably three colorants, more preferably the three primary iron oxide colorants, yellow, red and black, separately microencapsulated, and one or more microencapsulated or non-capsulated active agents selected from moistening, revitalizing/regenerating, antioxidant, and sunscreen agents;
(iv) a whitening/lightening skin care composition comprising one or more, preferably three colorants, more preferably the three primary iron oxide colorants, yellow, red and black separately microencapsulated, and one or more microencapsulated or non-capsulated active agents selected from moistening, revitalizing, antioxidant, and sunscreen agents;
(v) an aftershave composition comprising one or more, preferably three colorants, more preferably the three primary iron oxide colorants, yellow, red and black separately microencapsulated, and one or more optionally microencapsulated active agents selected from moistening, revitalizing/regenerating, cooling/calming, and anti-inflammatory agents;
(vi) a facemask comprising one or more separately microencapsulated, and one or more optionally microencapsulated active agent selected from a rejuvenating, an antioxidant, a astringent, a revitalizing/regenerating and a moistening agent;
(vii) an anti-acne composition comprising one or more colorants separately microencapsulated, and one or more optionally microencapsulated active agents selected from anti-acne therapeutic agents such as antibiotics, keratolytic, moistening, calming/cooling, antioxidant and sunscreen agents; or
(viii) an anti-cellulite composition comprising one or more, preferably three colorants, more preferably the three primary iron oxide colorants, yellow, red and black separately microencapsulated, and one or more optionally microencapsulated active agents selected from Rutin hydrate, moistening, antioxidant/free radical scavenger, sunscreen, astringent and revitalizing agents.

9. The color-changing composition according to any one of claims 1 to 8, in the form of cream, lotion, gel, milk, balm, mask or make-up personal care product.

## Patentansprüche

1. Farbänderungszusammensetzung zur Hautpflege, die für einen Farbänderungseffekt sorgt, welcher die Zufuhr von einem oder mehr Wirkstoffen zur Haut anzeigt, wobei die Zusammensetzung umfasst:
(i) einen oder mehr Wirkstoffe zur Hautpflege, die unter Mitteln zur Befeuchtung, Heilung/Regeneration/Revitalisierung, Depigmentierung/Aufhellung, Antioxidans/Radikalenfänger, Kühlung/Beruhigung, Erwärmung, Anästhetikum, Sonnenschutzmittel/Sonnenblocker, Antibiotika, Anticellulitemittel, Keratolytikum, Antimykotikum, Antipsoriatikum, Entzündungshemmer, antibakterielles Mittel, Adstringent, Antiseptikum, Repellent, Mittel gegen Besenreiser, Rosazea/Couperose, Aknemittel, Düfte/Parfums oder Mitteln zur Behandlung von Fieberbläschen ausgewählt werden; und
(ii) ein oder mehr mikroverkapselte Färbemittel;
wobei
ein oder mehrere mikroverkapselte Werbemittel einzeln oder als Mischung von Färbemitteln im Inneren von Doppel- oder vielschichtigen Mikrokapseln verkapselt sind, wobei das Innere, das das eine oder die mehreren Färbemittel enthält, von zwei oder mehr Hüllen aus demselben oder aus verschiedenen wandbildenden Polymeren umgeben ist, und
die Zusammensetzung sofort und unumkehrbar ihre Farbe beim Auftragen auf die Haut ändert.

2. Farbänderungszusammensetzung nach Anspruch 1, wobei die Färbemittel aus organischen Pigmenten, anorganischen Pigmenten, Laken, natürlichen und synthetischen Farbstoffen oder einer Kombination derselben ausgewählt werden.

3. Farbänderungszusammensetzung nach Anspruch 2, wobei die Färbemittel ausgewählt werden aus Metalloxiden, vorzugsweise Eisenoxiden, Titandioxid, Chromoxid, Zinkoxid und Verbundmetalloxiden; Metallhydroxiden, wie zum Beispiel Kalziumhydroxid, Eisenhydroxiden, Aluminiumhydroxid, Chromhydroxid, Magnesiumhydroxid und Verbundmetallhydroxiden; anderen Färbemitteln, wie zum Beispiel Eisen-Ammoniumferrocyanid, Preußischblau, Eisensulfide, Manganviolett, Carbon Black, Glimmer, Kaolin; organischen Lake-Pigmenten, wie zum Beispiel Indigo-Laken, Caimin-Laken, Laken aus der Reihe FD&C, und D&C-Farbstoffen, wie zum Beispiel D&C Red 21 Aluminum Lake, D&C Red 7 Calcium Lake; oder einem natürlichen oder synthetischen organischen Farbstoff, der aus aromatischen Azo-, Indigoid-, Triphenylmethan-, Anthrachinon- oder Xanthin-Farbstoffen der D&C- und FD&C-Reihe ausgewählt wird.

4. Farbänderungszusammensetzung nach einem der Ansprüche 1 bis 3, wobei der eine oder die mehreren Wirkstoffe zur Hautpflege in einer mikroverkapselten oder in nicht verkapselter Form sind, und beim Auftragen auf die Haut sorgt die Zusammensetzung für einen Farbänderungseffekt, der ein Anzeichen für die Zufuhr des einen oder der mehreren Wirkstoffe zum Auftragsbereich auf der Haut ist.

5. Farbänderungszusammensetzung nach Anspruch 1, wobei der eine oder die mehreren Wirkstoffe für die Hautpflege aus Folgendem ausgewählt werden:
(i) Befeuchtungs- oder Feuchthaltungsmittel, wie zum Beispiel, ohne darauf beschränkt zu sein, Nachtkerzenöl, Borretschsamenöl, Jojobaöl, Aloe vera-Gel, Vitamin F, Panthenol und Mischungen derselben;
(ii) Heil-/Revitalisierungs-/Regenerationsmittel, zum Beispiel, ohne darauf beschränkt zu sein, Hippophae-(Sanddorn)-Öl, Teebaumöl, Allantoin und Derivate, Carotinoide, Karottensamenextrakt und -öl, Patschuli-Aromaöl und Mischungen derselben;
(iii) Pigmententfernungs- (Aufhellungs-)Mittel, wie zum Beispiel, ohne darauf beschränkt zu sein, Süßholzwurzelextrakt (Glycyrrhiza glabra), Arbutin, Kojisäure, Hydrochinon, Vitamin C und Derivate, und Mischungen derselben;
(iv) Antioxidantien/freie Radikalfänger, zum Beispiel, ohne darauf beschränkt zu sein, Vitamin E, Tocotrienole, Tocopherole, Vitamin F, Vitamin C und Derivate derselben, Rutin, Resveratrol und Derivate derselben, Blattextrakt von Camellia sinensis (grüner Tee oder schwarzer Tee), Traubenkernextrakt, Nachtkerzenöl, Borretschsamenöl, Ingwer-Aromaöl, Kurkumin, Chitosan, und Mischungen derselben;
(v) Kühl- und Beruhigungsmittel, wie zum Beispiel, ohne darauf beschränkt zu sein, Menthol, Blattextrakt von Aloe barbadensis, Campher, Menthyllaktat, Allantoin, Bisabalol, Kamillenextrakt und Aromaöl, Nachtkerzenöl, Borretschsamenöl, Aromaöl von Eucalyptus citriodora, Patschuli-Aromaöl, Panthenol, und Mischungen derselben;
(vi) Erwärmungsmittel, wie zum Beispiel, ohne darauf beschränkt zu sein, Extrakt aus Schwarzpfeffer und Aromaöl, Paprika (Peperoni)-Extrakt, Zimtextrakt und -aromaöl, Ingwerwurzelextrakt und Aromaöl, Zeolith; und Mischungen derselben;
(vii) Anästhetika, wie zum Beispiel, ohne darauf beschränkt zu sein, Aloe barbadensis-Gel (Aloe vera-Gel), Benzocain, Lidocain, Dibucain, Pramoxin, Tetracain, Kampfer, Resorcinol, und Mischungen derselben;
(viii) Sonnenschutzmittel/Sonnenblocker, wie zum Beispiel, ohne darauf beschränkt zu sein, p-Aminobenzoesäure (PABA) und Ester derselben, Benzalphthalide, Benzophenone, Zimtsäureester (Cinnamate), Etocrylen, Octocrylen, Traubenkernextrakt, Titandioxid, Zinkoxid, und Mischungen derselben;
(ix) Antibiotika zur topischen Anwendung, wie zum Beispiel, ohne darauf beschränkt zu sein, Erythromycin, Clarithromycin, Azithromycin und Clindamycin;
(x) Anticellulitemittel, wie zum Beispiel, ohne darauf beschränkt zu sein, Rutinhydrat;
(xi) Keratolytika, die zur Behandlung von Akne, Warzen und anderen Hautkrankheiten nützlich sind, wie zum Beispiel, ohne darauf beschränkt zu sein, Benzoylperoxid, Schwefel, Aminolävulinsäure, Fluorouracil, Podophyllotoxin, Podophyllum, Propylenglycol, Phytinsäure, und Mischungen derselben;
(xii) Antibakterielle Mittel, wie zum Beispiel, ohne darauf beschränkt zu sein, Antibiotika, ätherisches Kamillenöl;
(xiii) Antimykotika, wie zum Beispiel, ohne darauf beschränkt zu sein, Teebaumöl, Traubenkernextrakt, ätherisches Eucalyptus citriodora-Öl, Ursolsäure, ätherische Öle, Amphotericin, Itraconazol, Fluconazol, Ketoconazol, Miconazol, Morpholin, Undecylensäure, und Mischungen derselben;
(xiv) Aknemittel, wie zum Beispiel, ohne darauf beschränkt zu sein, Benzoylperoxid, ätherisches Kamillenöl, und Mischungen derselben;
(xv) Antipsoriatika, wie zum Beispiel, ohne darauf beschränkt zu sein, Vitamin C, Vitamin D3 und Analoga derselben, Ergocalciferol, Anthralin/Cignolin, Metronidazol, Tazaroten, Cyclosporin, Pyrogallol, Allantoin, und Mischungen derselben;
(xvi) Entzündungshemmende Mittel, wie zum Beispiel, ohne darauf beschränkt zu sein, Vitamin F, Vitamin E, ungesättigte Fettsäuren, Rutin, Bioflavanoide, Hippophae- (Sanddorn)-Öl, Olivenöl, Salicin, Ingwerwurzelextrakt und ätherisches Öl, Jojobaöl, ätherisches Kamillenöl, ätherisches Eucalyptus citriodora-Öl, Ursolsäure, Triamcinolone, Cortisone, Prednisone, Cortodoxon, Flucetonid, Medryson, Amcinafel, Amcinafid, Betamethason und Ester derselben, Clocortelon, Descinolon, Desonid, Flucloronid, Flumethason, Flunisolid, Fluocinonid, Flucortolon, Paramethason, Dexamethason, Fluoroandrenolonacetonid, Acetonid, Dichlorison, und Mischungen derselben;
(xvii) Adstringierende Mittel, wie zum Beispiel, ohne darauf beschränkt zu sein, Zaubernuss (Hamamelis virginiana), Weiße Schafgarbe (Achillea millefolium), Palisanderöl, Benzoin, Aluminiumsulfat und andere Aluminiumsalze, Zinkoxid, und Mischungen derselben;
(xviii) Antiseptika, wie zum Beispiel, ohne darauf beschränkt zu sein, Honig, Kurkumin, Captan, Chlorhexidin und seine Derivate, Hexachlorophen, Triclosan, Triacetin, Natriumusnat, Schwefel, und Mischungen derselben;
(xix) Repellents, wie zum Beispiel, ohne darauf beschränkt zu sein, ätherische Öle, Pyrethrin, Permethrin, Bioresmethin, Dimethylphthalat, und Mischungen derselben;
(xx) Mittel gegen Rosacea, wie zum Beispiel, ohne darauf beschränkt zu sein, Vitamin K, Schwefel, Ringelblumenöl, Rutin, Bioflavanoide, und Mischungen derselben;
(xxi) Wirkstoffe, die nützlich zur lokalen Behandlung von Fieberbläschen sind (Bläschenausschlag und Gürtelrose), einschließlich, ohne darauf beschränkt zu sein, Aciclovir, Antihistamine, lokales Anästhetikum, ätherische Öle, die antivirale Aktivität besitzen, und Mischungen derselben; und
(xxii) Düfte/Parfums einschließlich, ohne darauf beschränkt zu sein, Lavendel, Neroli-Duftöl, ätherisches Kamillenöl, ätherisches Ingweröl, ätherisches Patschuliöl, ätherisches Öl von Eucalyptus citriodora, ätherisches Rosmarinöl, Sandelholzöl, Teebaumöl, und Mischungen derselben.

6. Farbänderungszusammensetzung nach einem der Ansprüche 1 bis 5, die als Öl-in-Wasser-, Öl-in-Wasser-in-Öl-, Wasser-in-Öl- oder Wasser-in-Öl-in Wasser-Emulsion, eine wässrige Formulierung oder eine wasserfreie Formulierung formuliert ist.

7. Kosmetische oder therapeutische Farbänderungszusammensetzung nach Anspruch 6, wobei die Zusammensetzung eine multifunktionale Zusammensetzung ist, die aus Körperpflege-, Gesichtspflege-, Babypflege-, Sonnenschutzpflege-, Pflege- nach dem Sonnenbad, Aufhellungs-, Pflege- nach der Rasur, Antiakne-, Repellent-, Anticellulite- und Antitranspirant-Zusammensetzungen ausgewählt wird.

8. Multifunktionale kosmetische Farbänderungszusammensetzung nach Anspruch 7, die ausgewählt wird:
(i) aus einer Sonnenschutz-/Sonnenblockerzusammensetzung, die ein oder mehr, vorzugsweise drei oder mehr Färbemittel umfasst, die aus Eisenoxiden, Titandioxid, oxidärmeres Titanoxid, Aluminiumoxid, Zirkoniumoxiden, Cobaltoxiden, Ceriumoxiden, Nickeloxiden oder Zinkoxid ausgewählt wird, oder aus zusammengesetzten Oxiden, vorzugsweise aus einem Eisenoxid, das aus rotem Eisenoxid, gelbem Eisenoxid oder schwarzem Eisenoxid ausgewählt wird, oder einer Mischung derselben, vorzugsweise getrennt mikroverkapselt, ausgewählt wird, und einen oder mehr mikroverkapselte oder nicht verkapselte Wirkstoffe umfasst, die aus Sonnenschutzmitteln, vorzugsweise sowohl organischen als auch anorganischen Sonnenschutzmitteln, Antioxidantien/Radikalenfängern und Befeuchtungsmitteln ausgewählt werden;
(ii) aus einer Zusammensetzung zum Gebrauch nach dem Sonnen/nach dem Bräunen, die ein oder mehr, vorzugsweise drei Färbemittel umfasst, besser die drei primären Eisenoxid-Färbemittel, gelb, rot und schwarz, getrennt mikroverkapselt, und einen oder mehr Wirkstoffe in einer mikroverkapselten oder nicht verkapselten Form, die aus Befeuchtungs-, Revitalisierungs-, Kühl-, Beruhigungs-, schmerzstillenden Mitteln oder Antioxidantien/Freiradikalfängern ausgewählt werden;
(iii) aus einer Anti-Aging-Gesichtscreme, die ein oder mehr, vorzugsweise drei Färbemittel umfasst, besser die drei primären Eisenoxid-Färbemittel, gelb, rot und schwarz, getrennt mikroverkapselt, und einen oder mehr mikroverkapselte oder nicht verkapselte Wirkstoffe, die aus Befeuchtungs-, Revitalisierungs-/Regenerationsmitteln, Antioxidantien und Sonnenschutzmitteln ausgewählt werden;
(iv) aus einer Aufhellungs-Hautpflegezusammensetzung, die ein oder mehr, vorzugsweise drei Färbemittel umfasst, besser die drei primären Eisenoxid-Färbemittel, gelb, rot und schwarz, getrennt mikroverkapselt, und einen oder mehr Wirkstoffe in einer mikroverkapselten oder nicht verkapselten Form umfasst, die aus Befeuchtungs-, Revitalisierungsmitteln, Antioxidantien und Sonnenschutzmitteln ausgewählt werden;
(v) aus einer Aftershave-Zusammensetzung, die ein oder mehr, vorzugsweise drei Färbemittel umfasst, besser die drei primären Eisenoxid-Färbemittel, gelb, rot und schwarz, getrennt mikroverkapselt, und einen oder mehr optional mikroverkapselte Wirkstoffe umfasst, die aus Befeuchtungs-, Revitalisierungs-/Regenerierungsmitteln, Kühl-/Beruhigungsmitteln und einem entzündungshemmenden Mittel ausgewählt werden;
(vi) aus einer Gesichtsmaske, die einen oder mehr getrennt mikroverkapselte und einen oder mehr optional mikroverkapselte Wirkstoffe umfasst, die aus einem Verjüngungsmittel, einem Antioxidans, einem Adstringens, einem Belebungs-/Regenerierungsmittel und einem Befeuchtungsmittel ausgewählt werden;
(vii) aus einer Anti-Akne-Zusammensetzung, die ein oder mehr Färbemittel, die getrennt mikroverkapselt sind, und einen oder mehr optional mikroverkapselte Wirkstoffe umfasst, die aus Anti-Akne-Therapeutika ausgewählt werden, wie zum Beispiel Antibiotika, Keratolytika, Befeuchtungs-, Beruhigungs-/Kühlmittel, Antioxidantien und Sonnenschutzmittel; oder
(viii) aus einer Anti-Cellulite-Zusammensetzung, die ein oder mehr, vorzugsweise drei Färbemittel umfasst, besser die drei primären Eisenoxid-Färbemittel, gelb, rot und schwarz, getrennt mikroverkapselt, und einen oder mehr optional mikroverkapselte Wirkstoffe umfasst, die aus Rutinhydrat, Befeuchtungsmittel, Antioxidantien/Freiradikalfängern, Sonnenschutzmittel, Adstringentien und Revitalisierungsmitteln ausgewählt werden.

9. Farbänderungszusammensetzung nach einem der Ansprüche 1 bis 8, in Form von Creme, Lotion, Gel, Milch, Balsam, Maske oder Make-up-Pflegeprodukt.

## Revendications

1. Composition à changement de couleur pour le soin de la peau, qui produit un effet de changement de couleur indiquant la distribution d'une ou de plusieurs substances actives sur la peau, la composition comprenant :
(i) une ou plusieurs substances actives pour le soin de la peau choisies parmi les agents hydratants, cicatrisants/régénérants/revitalisants, dépigmentants/blanchissants/éclaircissants, antioxydants/piégeurs de radicaux libres, rafraîchissants/apaisants, chauffants, anesthésiants, filtrant/bloquant les rayons solaires, antibiotiques, anticellulite, kératolytiques, antifongiques, antipsoriasiques, anti-inflammatoires, antibactériens, astringents, antiseptiques, répulsifs, anti-dilatation des petits vaisseaux sanguins, anti-rosacée/anti-couperose, anti-acnéiques, les fragrances/les parfums ou les agents destinés au traitement des boutons de fièvre ; et
(ii) un ou plusieurs colorants micro-encapsulés ;
dans laquelle
lesdits un ou plusieurs colorants micro-encapsulés sont encapsulés individuellement ou sous la forme d'un mélange de colorants dans le coeur des microcapsules à double couche ou multicouche, ledit coeur contenant les un ou plusieurs colorants entourés par deux ou plusieurs enveloppes du même polymère ou d'un autre polymère formant la paroi, et
ladite composition change immédiatement et de manière irréversible de couleur lors de l'application sur la peau.

2. Composition à changement de couleur selon la revendication 1, dans laquelle les colorants sont choisis parmi les pigments organiques, les pigments inorganiques, les laques, les colorants naturels et synthétiques ou toute combinaison de ceux-ci.

3. Composition à changement de couleur selon la revendication 2, dans laquelle lesdits colorants sont choisis parmi les oxydes métalliques, de préférence les oxydes de fer, le dioxyde de titane, l'oxyde de chrome, l'oxyde de zinc et les oxydes de métaux composites ; les hydroxydes métalliques comme l'hydroxyde de calcium, les hydroxydes de fer, l'hydroxyde d'aluminium, l'hydroxyde de chrome, l'hydroxyde de magnésium et les hydroxydes de métaux composites ; les autres colorants comme le ferrocyanure d'ammonium ferrique, le bleu de Prusse, les sulfures de fer, le violet de manganèse, le noir de carbone, le mica, le kaolin ; les pigments organiques laques comme les laques Indigo, les laques Carmin, les laques provenant des colorants des séries FD&C et D&C comme la laque aluminium Red 21 de D&C, la laque calcium Red 7 de D&C ; ou un colorant organique naturel ou synthétique choisi parmi les colorants azoïques aromatiques, indigoïdes, triphénylméthane, anthraquinone ou xanthine des séries D&C et FD&C.

4. Composition à changement de couleur selon l'une quelconque des revendications 1 à 3, dans laquelle les une ou plusieurs substances actives pour le soin de la peau sont sous une forme micro-encapsulée ou non encapsulée, et lors de l'application sur la peau, la composition produit un effet de changement de couleur qui est un indicateur de la distribution de la une ou plusieurs substances actives sur la zone d'application de la peau.

5. Composition à changement de couleur selon la revendication 1, dans laquelle les une ou plusieurs substances actives pour le soin de la peau sont choisies parmi :
(i) les agents hydratants ou humectants tels que, mais sans y être limité, l'huile d'onagre, l'huile de bourrache, l'huile de jojoba, le gel d'Aloe vera, la vitamine F, le panthénol et des mélanges de ceux-ci ;
(ii) les agents cicatrisants/revitalisants/régénérants tels que, mais sans y être limité, l'huile d'argousier (hippophae), l'huile d'arbre à thé (tea tree), l'allantoïne et ses dérivés, les caroténoïdes, l'extrait et l'huile de graines de carotte, l'huile essentielle de patchouli, et des mélanges de ceux-ci ;
(iii) les agents dépigmentants (blanchissants)/éclaircissants tels que, mais sans y être limité, l'extrait de racine de réglisse (Glycyrrhiza Glabray l'arbutine, l'acide kojique, l'hydroquinone, la vitamine C et ses dérivés, et des mélanges de ceux-ci ;
(iv) les agents antioxydants/piégeurs de radicaux libres tels que, mais sans y être limité, la vitamine E, les tocotriénols, les tocophérols, la vitamine F, la vitamine C et leurs dérivés, la rutine, le resvératrol et ses dérivés, l'extrait de feuilles de thé vert ou de thé noir (Camellia Sinensis), l'extrait de pépin de raisin, l'huile d'onagre, l'huile de bourrache, l'huile essentielle de gingembre, la curcumine, le chitosan, et des mélanges de ceux-ci ;
(v) les agents rafraîchissants et apaisants tels que, mais sans y être limité, le menthol, l'extrait de feuilles d'Aloe Barbadensis, le camphre, le menthyl lactate, l'allantoïne, le bisabalol, l'extrait et l'huile essentielle de camomille, l'huile d'onagre, l'huile de bourrache, l'huile essentielle d'eucalyptus citronné, l'huile essentielle de patchouli, le panthénol et des mélanges de ceux-ci ;
(vi) les agents chauffants tels que, mais sans y être limité, l'extrait et l'huile essentielle de poivre noir, l'extrait de paprika (poivre rouge), l'extrait et l'huile essentielle de cannelle, l'extrait et l'huile essentielle de racine de gingembre, la zéolithe ; et des mélanges de ceux-ci ;
(vii) les agents anesthésiants tels que, mais sans y être limité, le gel d'Aloe barbadensis (le gel d'Aloe vera), la benzocaïne, la lidocaïne, la dibucaïne, la pramoxine, la tétracaïne, le camphre, le résorcinol, et des mélanges de ceux-ci ;
(viii) les agents filtrant/bloquant les rayons solaires tels que, mais sans y être limité, l'acide p-aminobenzoïque (PABA) et les esters de celui-ci, les benzalphtalides, les benzophénones, les cinnamates, l'étocrylène, l'octocrylène, l'extrait de pépins de raisin, le dioxyde de titane, l'oxyde de zinc, et des mélanges de ceux-ci ;
(ix) les antibiotiques pour une application topique tels que, mais sans y être limité, l'érythromycine, la clarithromycine, l'azithromycine et la clindamycine ;
(x) les agents anticellulite tels que, mais sans y être limité, l'hydrate de rutine ;
(xi) les agents kératolytiques utiles pour le traitement de l'acné, des verrues et autres maladies de la peau, tels que, mais sans y être limité, le peroxyde de benzoyle, le soufre, l'acide aminolévulinique, le fluorouracile, la podophyllotoxine, le podophyllum, le propylène glycol, l'acide phytique et des mélanges de ceux-ci ;
(xii) les agents antibactériens tels que, mais sans y être limité, les antibiotiques, l'huile essentielle de camomille ;
(xiii) les agents antifongiques tels que, mais sans y être limité, l'huile d'arbre à thé, l'extrait de pépins de raisin, l'huile essentielle d'eucalyptus citronné, l'acide ursolique, les huiles essentielles, l'amphotéricine, l'itaconazole, le fluconazole, le kétoconazole, le miconazole, la morpholine, l'acide undécylénique, et des mélanges de ceux-ci ;
(xiv) les agents anti-acnéiques tels que, mais sans y être limité, le peroxyde de benzoyle, l'huile essentielle de camomille et des mélanges de ceux-ci ;
(xv) les agents antipsoriasiques tels que, mais sans y être limité, la vitamine C, la vitamine D3 et des analogues de celles-ci, l'ergocalciférol, l'anthraline, le métronidazole, le tazarotène, la cyclosporine, le pyrogallol, l'allantoïne, et des mélanges de ceux-ci ;
(xvi) les agents anti-inflammatoires tels que, mais sans y être limité, la vitamine F, la vitamine E, les acides gras insaturés, la rutine, les bioflavonoïdes, l'huile d'argousier (hippophae), l'huile d'olive, la salicine, l'extrait et l'huile essentielle de racine de gingembre, l'huile de jojoba, l'huile essentielle de camomille, l'huile essentielle d'eucalyptus citronné, l'acide ursolique, les triamcinolones, les cortisones, les prédnisones, la cortodoxone, le flucétonide, la médrysone, l'amcinafel, l'amcinafide, la bétaméthasone, et les esters de celle-ci, la clocortélone, la descinolone, le désonide, le flucloronide, la fluméthasone, le flunisolide, le fluocinonide, la flucortolone, la paraméthasone, la déxaméthasone, le fluorandrénolone acétonide, l'acétonide, la dichlorisone et des mélanges de ceux-ci ;
(xvii) les agents astringents tels que, mais sans y être limité, l'hamamélis virginiana, l'achillée millefeuilles, l'huile de palissandre, le benjoin, le sulfate d'aluminium et les autres sels d'aluminium, l'oxyde de zinc et des mélanges de ceux-ci ;
(xviii) les agents antiseptiques tels que, mais sans y être limité, le miel, la curcumine, le captane, la chlorhexidine et ses dérivés, l'hexachlorophène, le triclosan, la triacétine, l'usnate de sodium, le soufre et des mélanges de ceux-ci ;
(xix) les agents répulsifs tels que, mais sans y être limité, les huiles essentielles, la pyréthrine, la perméthrine, la bioresméthine, le diméthylphtalate et des mélanges de ceux-ci ;
(xx) les agents anti-rosacée tels que, mais sans y être limité, la vitamine K, le soufre, l'huile de fleur de souci, la rutine, les bioflavonoïdes et des mélanges de ceux-ci ;
(xxi) les substances actives bénéfiques pour le traitement local des boutons de fièvre (herpès labiaux et zonas) y compris, mais sans y être limité, l'acyclovir, les antihistamines, les anesthésiques locaux, les huiles essentielles possédant une activité antivirale, et des mélanges de ceux-ci ; et
(xxii) les fragrances/parfums y compris, mais sans y être limité, la lavande, l'essence de parfum de néroli, l'huile essentielle de camomille, l'huile essentielle de gingembre, l'huile essentielle de patchouli, l'huile essentielle d'eucalyptus citronné, l'huile essentielle de romarin, l'huile essentielle de bois de santal, l'huile d'arbre à thé, et des mélanges de ceux-ci.

6. Composition à changement de couleur selon l'une quelconque des revendications 1 à 5, formulée sous forme d'émulsion huile-dans-eau, huile-dans-eau-dans-huile, eau-dans-huile ou eau-dans-huile-dans-eau, de formulation aqueuse ou de formulation anhydre.

7. Composition cosmétique ou thérapeutique à changement de couleur selon la revendication 6, dans laquelle ladite composition multifonctionnelle est choisie parmi les compositions de soin de la peau du corps, de soin de la peau du visage, de soin pour bébé, de soin de protection solaire, d'après-soleil, de blanchissantes, d'après-rasage, d'anti-acnéiques, de répulsives, d'anti-cellulite et d'anti-perspirantes.

8. Composition cosmétique multifonctionnelle à changement de couleur selon la revendication 7, choisie parmi :
(i) une composition filtrant/bloquant les rayons solaires comprenant un ou plusieurs, de préférence trois colorants ou plus, choisis parmi les oxydes de fer, le dioxyde de titane, les oxydes inférieurs de titane, l'oxyde d'aluminium, les oxydes de zirconium, les oxydes de cobalt, les oxydes de cérium, les oxydes de nickel, ou l'oxyde de zinc, ou des oxydes composites, de préférence un oxyde de fer choisi parmi l'oxyde de fer rouge, l'oxyde de fer jaune ou l'oxyde de fer noir, ou un mélange de ceux-ci, de préférence micro-encapsulés séparément, et un ou plusieurs agents actifs micro-encapsulés ou non encapsulés choisis parmi les agents filtrant les rayons solaires, de préférence à la fois les agents filtrant les rayons solaires organiques et inorganiques, les agents antioxydants/piégeurs de radicaux libres et les agents hydratants ;
(ii) une composition après-soleil/après bronzage comprenant un ou plusieurs, de préférence trois colorants, plus préférentiellement les trois colorants primaires d'oxyde de fer, jaune, rouge et noir, micro-encapsulés séparément, et un ou plusieurs agents actifs sous une forme micro-encapsulée ou non encapsulée choisis parmi les agents hydratants, revitalisants, rafraîchissants, apaisants, anesthésiants ou antioxydants/piégeurs de radicaux libres ;
(iii) une crème anti-âge pour le visage comprenant un ou plusieurs, de préférence trois colorants, plus préférentiellement les trois colorants primaires d'oxyde de fer, jaune, rouge et noir, micro-encapsulés séparément, et un ou plusieurs agents actifs micro-encapsulés ou non encapsulés choisis parmi les agents hydratants, revitalisants/régénérants, antioxydants et les agents filtrant les rayons solaires ;
(iv) une composition de soin de la peau blanchissante/éclaircissante comprenant un ou plusieurs, de préférence trois colorants, plus préférentiellement les trois colorants primaires d'oxyde de fer, jaune, rouge et noir, micro-encapsulés séparément, et un ou plusieurs agents actifs micro-encapsulés ou non encapsulés choisis parmi les agents hydratants, revitalisants, antioxydants et les agents filtrant les rayons solaires ;
(v) une composition après-rasage comprenant un ou plusieurs, de préférence trois colorants, plus préférentiellement les trois colorants primaires d'oxyde de fer, jaune, rouge et noir, micro-encapsulés séparément, et un ou plusieurs agents actifs éventuellement micro-encapsulés choisis parmi les agents hydratants, revitalisants/régénérants, rafraîchissants/apaisants, et anti-inflammatoires ;
(vi) un masque pour le visage comprenant un ou plusieurs colorants micro-encapsulés séparément et un ou plusieurs agents actifs éventuellement micro-encapsulés choisis parmi un agent rajeunissant, un antioxydant, un astringent, un agent revitalisant/régénérant et un agent hydratant ;
(vii) une composition anti-acnéique comprenant un ou plusieurs colorants micro-encapsulés séparément et un ou plusieurs agents actifs éventuellement micro-encapsulés choisis parmi les agents thérapeutiques anti-acnéiques tels que les antibiotiques, les kératolytiques, les agents hydratants, les agents apaisants/rafraîchissants, les antioxydants et les agents filtrant les rayons solaires ; ou
(viii) une composition anti-cellulite comprenant un ou plusieurs, de préférence trois colorants, plus préférentiellement les trois colorants primaires d'oxyde de fer, jaune, rouge et noir, micro-encapsulés séparément, et un ou plusieurs agents actifs éventuellement micro-encapsulés choisis parmi l'hydrate de rutine, les agents hydratants, antioxydants/piégeurs de radicaux libres, les agents filtrant les rayons solaires, les agents astringents et revitalisants.

9. Composition à changement de couleur selon l'une quelconque des revendications 1 à 8, sous forme de crème, lotion, gel, lait, baume, masque ou maquillage pour l'hygiène personnelle.
